# EUROPEAN PATENT APPLICATION

(11) **EP 4 796 085 A2**
(43) Date of publication of application: **26.08.2026**
(21) Application number: 26188575.0
(22) Date of filing: 24.09.2021
(51) Int. Cl.: A61F 2/28

(54) **BONE IMPLANT WITH POROUS MEMBRANE AND METHOD FOR PREPARATION THEREOF**

(30) Priority: 25.09.2020 CN 202011025357
(62) Document number(s) of the earlier application(s) in accordance with Art. 76 EPC: 21871612.4 / 4 218 841
(71) Applicant: Beijing Huayu Chuangxin Technologies Co., Ltd., Beijing 101407 (CN); Beijing Huayu Chuangxin TA-NB Science & Technology Co., Ltd., Beijing 101407 (CN)
(72) Inventor: LIU, Yuzhong, Beijing, 101407 (CN)
(74) Representative: Herzog IP Patentanwalts GmbH

(57) **Abstract**

The present invention relates to a bone implant with a porous lithium tantalate membrane and a method for preparing the bone implant. The bone implant comprises: (1) a substrate; and (2) a porous membrane on the substrate, wherein the substrate is selected from the group consisting of a tantalum substrate, a niobium substrate, a tantalum-niobium alloy substrate and a titanium substrate, and wherein the porous membrane is selected from the group consisting of a porous lithium tantalate membrane, a porous lithium niobate membrane, a porous lithium tantalate-lithium niobate mixture membrane and a porous titanium oxide membrane. The bone implant of the present invention has one or more of the following beneficial effects: (1) The bone implant has excellent corrosion resistance; (2) the elasticity modulus of the bone implant can be adjusted according to process conditions so that it has higher biocompatibility with the elasticity modulus of a human or animal bone (such as an alveolar bone and a cranium); (3) the white color of the bone implant is close to the color of the bone itself and the bone implant has an aesthetic appearance; (4) the bone implant has excellent bacteriostatic properties.

## Description

### Technical Field

The present invention relates to a bone implant with a porous membrane and a method for preparing the bone implant.

### Background Art

Metal materials have excellent comprehensive mechanical properties and anti-fatigue properties and are particularly suitable for use as orthopedic implant materials. With the rapid development of materials science, orthopedic implant materials have experienced the development process from stainless steel, cobalt-chromium alloys to titanium alloys and achieved relatively good effects. However, complicated environments inside human bodies cause material corrosion, which results in a release of toxic elements and further a reduction in biocompatibility of some metal materials. In addition, materials in the prior art, such as stainless steel, cobalt-chromium alloys and titanium alloys, have a relatively dark color or even are black, which affects the aesthetic properties of the materials and limits the scope of the applications. The above deficiencies have certain negative impacts on the application of the metal materials as biomedical materials.

Porous tantalum is a relatively ideal orthopedic implant material that has emerged in recent years. As a refractory metal, it has a melting point of nearly 3000°C, is dark gray in appearance and has a smooth surface. Compared with existing medical metal materials, tantalum has two main advantages: (1) tantalum has more excellent corrosion resistance; (2) tantalum has better biocompatibility and porous tantalum has a relatively low elasticity modulus. However, due to its dark gray color, tantalum affects aesthetic appearance when used particularly in dental implants.

Porous tantalum is a relatively ideal orthopedic implant material, and documented orthopedic materials made of tantalum have an elasticity modulus between the elasticity modulus of cortical bone and the elasticity modulus of cancellous bone. However, the elasticity modulus of porous tantalum is closer to that of human cancellous bone, but significantly lower than that of cortical bone. Moreover, a bone tissue that bears the load of a human body is mainly cortical bone. From the perspective of animal experiments and clinical application effects, the elasticity modulus of bone implants should be closer to that of human cortical bone; a too high elasticity modulus makes it easy to produce stress shielding while a too low elasticity modulus is not conducive to force transmission. The present invention can provide elasticity modulus from low to high for selection under different coating conditions.

However, with regard to dental implants, from a biomechanical point of view, the elasticity modulus of a dental implant has an effect on stress distribution at a bone interface. Generally, the higher the elasticity modulus of a dental implant, the smaller the internal stress of the bone around the neck and the greater the internal stress of the bone at the root; the lower the elasticity modulus of a dental implant, the greater the relative displacement motion of the dental implant and the bone interface. When the elasticity modulus of a dental implant is similar to that of cortical bone, cancellous bone, the biomechanical compatibility is relatively good. Some scholars have suggested that a high elasticity modulus is better and that an elasticity modulus suitable for a dental implant is preferably 70 to 200 GPa, and it is reported that the elasticity modulus of cortical bone is about 18 GPa.

However, there are different requirements for the elasticity modulus of cranium (skull) implants.

Dental implants are made of titanium alloys or other materials into cylinders or cones, on the outer surfaces of which threads are formed, with an aim that human bones can grow into the threads, which allows the implants to integrate with the human bones and allows for the enhanced compatibility and firmness of the implants and the human bodies. Nevertheless, threaded ribs are limited in depth, and an excessive depth affects the strength of the implants.

Therefore, there is still a need to seek a bone implant with aesthetic appearance and/or higher biocompatibility, particularly a dental implant and a cranium (skull) implant.

### Contents of Invention

For this purpose, the present invention provides a bone implant or complex comprising:
(1) a substrate; and
(2) a porous membrane on the substrate,

wherein the substrate is selected from the group consisting of a tantalum substrate, a niobium substrate, a tantalum-niobium alloy substrate and a titanium substrate, and
wherein the porous membrane is selected from the group consisting of a porous lithium tantalate membrane, a porous lithium niobate membrane, a porous lithium tantalate-lithium niobate mixture membrane and a porous titanium oxide membrane.

Further, the present invention provides a bone implant or complex comprising:
(1) a tantalum substrate, a niobium substrate, a tantalum-niobium alloy substrate or a titanium substrate; and
(2) a porous lithium tantalate membrane on the tantalum substrate, wherein the lithium tantalate membrane contains a tantalum oxide,

a porous lithium niobate membrane on the niobium substrate, wherein the lithium niobate membrane contains a niobium oxide,
a porous lithium tantalate-lithium niobate mixture membrane on the tantalum-niobium alloy substrate, wherein the mixture membrane contains a tantalum oxide and a niobium oxide, or a porous titanium oxide membrane on the titanium substrate.

The present invention also provides a method for preparing the bone implant or complex, comprising:
(1) providing a substrate; and
(2) forming a porous membrane on the substrate,

wherein the substrate is selected from the group consisting of a tantalum substrate, a niobium substrate, a tantalum-niobium alloy substrate and a titanium substrate, and
wherein the porous membrane is selected from the group consisting of a porous lithium tantalate membrane, a porous lithium niobate membrane, a porous lithium tantalate-lithium niobate mixture membrane and a porous titanium oxide membrane.

Further, the present invention provides a method for preparing the bone implant or complex, comprising:
(1) providing a tantalum substrate, a niobium substrate, a tantalum-niobium alloy substrate or a titanium substrate; and
(2) forming a porous lithium tantalate membrane, a porous lithium niobate membrane, or a porous lithium tantalate-lithium niobate mixture membrane on the tantalum substrate, niobium substrate or tantalum-niobium alloy substrate, or forming a porous titanium oxide membrane on the titanium substrate,

wherein the lithium tantalate membrane contains a tantalum oxide,
wherein the lithium niobate membrane contains a niobium oxide, and
wherein the mixture membrane contains a tantalum oxide and a niobium oxide.

The present invention also relates to a use of the complex in medical materials, such as dental implants.

The bone implant of the present invention has one or more of the following beneficial effects: (1) The metal compound membrane provided is porous, which is conducive to guiding a human bone into the implant and enhancing the compatibility and firmness of the implant. Meanwhile, the compound membrane layer seems to be close to a ceramic body, which has the effect of insulation or heat insulation and reduces the stimulation of rapid heat to a human body. (2) The membrane layer is not formed by ion deposition in a solution, but results from the participation of the metal of the implant in an electrochemical reaction; there is a performance transition layer; the membrane layer is firmly bonded to the substrate, and the bone implant has excellent corrosion resistance. (3) The elasticity modulus of the bone implant can be adjusted according to process conditions so that it has higher biocompatibility with the elasticity modulus of a human or animal bone (such as an alveolar bone). (4) The white color of the bone implant is close to the color of the bone itself and the bone implant has an aesthetic appearance. (5) The bone implant has excellent bacteriostatic properties since the membrane layer results from coating treatment at high temperatures (above 400°C).

### Description of Figures

Figures 1a-1c show SEM photographs of a pure tantalum flake not coated with a porous lithium tantalate membrane.
Figures 2a-2d show SEM photographs of a tantalum flake coated with a porous lithium tantalate membrane according to one embodiment of the present invention.
Figures 3a-3c show SEM photographs of a tantalum flake coated with a porous lithium tantalate membrane according to one embodiment of the present invention.
Figures 4a-4c show SEM photographs of a tantalum flake coated with a porous lithium tantalate membrane according to one embodiment of the present invention.
Figures 5a-5c show SEM photographs of a tantalum flake coated with a porous lithium tantalate membrane according to one embodiment of the present invention.
Figures 6a-6c show SEM photographs of a tantalum flake coated with a porous lithium tantalate membrane according to one embodiment of the present invention.
Figures 7a-7d show SEM photographs of a tantalum flake coated with a porous lithium tantalate membrane according to one embodiment of the present invention.
Figures 8a-8c show SEM photographs of a tantalum flake coated with a porous lithium tantalate membrane according to one embodiment of the present invention.
Figure 9 shows an appearance photograph of a tantalum flake coated with a porous lithium tantalate membrane according to one embodiment of the present invention.
Figure 10 shows an appearance photograph of a tantalum flake coated with a porous lithium tantalate membrane according to one embodiment of the present invention.
Figure 11 shows an appearance photograph of a tantalum flake coated with a porous lithium tantalate membrane according to one embodiment of the present invention.
Figure 12 shows an appearance photograph of a tantalum flake coated with a porous lithium tantalate membrane according to one embodiment of the present invention.
Figure 13 shows an appearance photograph of a tantalum flake and a tantalum dental implant coated with a porous lithium tantalate membrane according to one embodiment of the present invention.
Figure 14 shows an appearance photograph of a tantalum flake and a tantalum dental implant coated with a porous lithium tantalate membrane according to one embodiment of the present invention.
Figure 14a shows an appearance photograph of a porous tantalum dental implant coated with a porous lithium tantalate membrane according to one embodiment of the present invention.
Figures 15a-15g show appearance photographs of a tantalum flake coated with a porous lithium tantalate membrane according to one embodiment of the present invention.
Figures 16a-16b show SEM photographs of a tantalum flake coated with a porous lithium tantalate membrane according to one embodiment of the present invention.
Figures 17a-17b show SEM photographs of a tantalum flake coated with a porous lithium tantalate membrane according to one embodiment of the present invention.
Figures 18a-18b show SEM photographs of a tantalum flake coated with a porous lithium tantalate membrane according to one embodiment of the present invention.
Figures 19a-19b show SEM photographs of a tantalum flake coated with a porous lithium tantalate membrane according to one embodiment of the present invention.
Figures 20a-20b show SEM photographs of a tantalum flake coated with a porous lithium tantalate membrane according to one embodiment of the present invention.
Figures 21a-21b show SEM photographs of a tantalum flake coated with a porous lithium tantalate membrane according to one embodiment of the present invention.
Figures 22a-22b show SEM photographs of a tantalum flake coated with a porous lithium tantalate membrane according to one embodiment of the present invention.
Figures 23a-23i show appearance photographs of a tantalum flake coated with a porous lithium tantalate membrane according to one embodiment of the present invention.
Figures 24a-24b show SEM photographs of a tantalum flake coated with a porous lithium tantalate membrane according to one embodiment of the present invention.
Figures 25a-25b show SEM photographs of a tantalum flake coated with a porous lithium tantalate membrane according to one embodiment of the present invention.
Figures 26a-26b show SEM photographs of a tantalum flake coated with a porous lithium tantalate membrane according to one embodiment of the present invention.
Figures 27a-27b show SEM photographs of a tantalum flake coated with a porous lithium tantalate membrane according to one embodiment of the present invention.
Figures 28a-28b show SEM photographs of a tantalum flake coated with a porous lithium tantalate membrane according to one embodiment of the present invention.
Figures 29a-29b show SEM photographs of a tantalum flake coated with a porous lithium tantalate membrane according to one embodiment of the present invention.
Figures 30a-30b show SEM photographs of a tantalum flake coated with a porous lithium tantalate membrane according to one embodiment of the present invention.
Figures 31a-31b show SEM photographs of a tantalum flake coated with a porous lithium tantalate membrane according to one embodiment of the present invention.
Figures 32a-32b show SEM photographs of a tantalum flake coated with a porous lithium tantalate membrane according to one embodiment of the present invention.
Figure 33a shows an appearance photograph of a tantalum cranium coated with a porous lithium tantalate membrane according to one embodiment of the present invention.
Figure 33b shows a schematic cross-sectional diagram of a tantalum cranium coated with a porous lithium tantalate membrane according to one embodiment of the present invention.
Figure 33c shows a schematic top view diagram of a tantalum cranium coated with a porous lithium tantalate membrane according to one embodiment of the present invention.
Figure 34 shows an appearance photograph of a tantalum-niobium alloy flake coated with a porous lithium tantalate-lithium niobate mixture membrane according to one embodiment of the present invention.
Figures 35a-35b show SEM photographs of a tantalum-niobium alloy flake coated with a porous lithium tantalate-lithium niobate mixture membrane according to one embodiment of the present invention.
Figure 36 shows an appearance photograph of a tantalum-niobium alloy flake coated with a porous lithium tantalate-lithium niobate mixture membrane according to one embodiment of the present invention.
Figures 37a-37b show SEM photographs of a tantalum-niobium alloy flake coated with a porous lithium tantalate-lithium niobate mixture membrane according to one embodiment of the present invention.
Figure 38 shows an appearance photograph of a niobium flake coated with a porous lithium niobate membrane according to one embodiment of the present invention.
Figures 39a-39b show SEM photographs of a niobium flake coated with a porous lithium niobate membrane according to one embodiment of the present invention.
Figure 40 shows an appearance photograph of a niobium flake coated with a porous lithium niobate membrane according to one embodiment of the present invention.
Figure 41 shows an appearance photograph of a niobium cranium coated with a porous lithium niobate membrane according to one embodiment of the present invention.
Figures 42a-42b show SEM photographs of a niobium flake coated with a porous lithium niobate membrane according to one embodiment of the present invention.
Figure 43 shows an appearance photograph of a niobium flake coated with a porous lithium niobate membrane according to one embodiment of the present invention.
Figures 44a-44b show SEM photographs of a niobium flake coated with a porous lithium niobate membrane according to one embodiment of the present invention.

### Specific Embodiments

In one embodiment, the present invention provides a bone implant or a tantalum complex comprising:
(1) a tantalum substrate; and
(2) a porous lithium tantalate membrane on the tantalum substrate, wherein the lithium tantalate membrane contains a tantalum oxide.

In one embodiment, the present invention provides a bone implant or a niobium complex comprising:
(1) a niobium substrate; and
(2) a porous lithium niobate membrane on the niobium substrate, wherein the lithium niobate membrane contains a niobium oxide.

In one embodiment, the present invention provides a bone implant or a tantalum-niobium alloy complex comprising:
(1) a tantalum-niobium alloy substrate; and
(2) a porous lithium tantalate-lithium niobate mixture membrane on the tantalum-niobium alloy substrate, wherein the mixture membrane contains a tantalum oxide and a niobium oxide.

In one embodiment, the present invention provides a bone implant or a titanium complex comprising:
(1) a titanium substrate; and
(2) a porous titanium oxide membrane on the titanium substrate.

In one embodiment, the bone implant or tantalum complex comprises: (1) a tantalum substrate; and (2) a porous lithium tantalate membrane on at least a portion of the surface of the tantalum substrate, wherein the porous lithium tantalate membrane has a pore diameter of 0.1-1 µm. For crack-like pores, the length thereof can reach about 40 µm.

In one embodiment, the bone implant or niobium complex comprises: (1) a niobium substrate; and (2) a porous lithium niobate membrane on at least a portion of the surface of the niobium substrate, wherein the porous lithium niobate membrane has a pore diameter of 0.1-1 µm. For crack-like pores, the length thereof can reach about 40 µm.

In one embodiment, the bone implant or tantalum-niobium alloy complex comprises: (1) a tantalum-niobium alloy substrate; and (2) a porous lithium tantalate-lithium niobate mixture membrane on at least a portion of the surface of the tantalum-niobium alloy substrate, wherein the mixture membrane has a pore size of 0.1-1 µm. For crack-like pores, the length thereof can reach about 40 µm.

In one embodiment, the bone implant or titanium complex comprises: (1) a titanium substrate; and (2) a porous titanium oxide membrane on at least a portion of the surface of the titanium substrate, wherein the porous titanium oxide membrane has a pore diameter of 0.1-1 µm. For crack-like pores, the length thereof can reach about 40 µm.

In the context of the present invention, the pores are not limited to round pores, but may be pores with irregular shapes, e.g., crack-like pores.

In one embodiment of the present invention, the bone implant or complex has a porosity of 30% or more, such as a porosity of 50-80% or a porosity of 60-90%.

In the context of the present invention, the bone implant, complex and composite may be used interchangeably.

Examples of the tantalum substrate suitable for the present invention include a pure tantalum substrate and a tantalum alloy substrate, wherein the tantalum alloy substrate is preferably a tantalum-niobium alloy substrate.

In one embodiment of the present invention, the bone implant, such as a dental implant, has an elasticity modulus of at least 150 GPa.

In another embodiment of the present invention, the bone implant, such as a dental implant, has an elasticity modulus of at least 170 GPa.

In yet another embodiment of the present invention, the bone implant, such as a dental implant, has an elasticity modulus of 150-200 GPa.

In still another embodiment of the present invention, the bone implant, such as a dental implant, has an elasticity modulus of 170-190 GPa.

In one embodiment of the present invention, the bone implant, such as a cranium (skull) implant, has an elasticity modulus of 10-160 GPa, as measured by nanoindentation.

In another embodiment of the present invention, the bone implant, such as a cranium (skull) implant, has an elasticity modulus of 20-150 GPa, as measured by nanoindentation.

In yet another embodiment of the present invention, the bone implant, such as a cranium (skull) implant, has an elasticity modulus of 30-140 GPa, as measured by nanoindentation.

In yet another embodiment of the present invention, the bone implant, such as a cranium (skull) implant, has an elasticity modulus of 40-130 GPa, as measured by nanoindentation.

In still another embodiment of the present invention, the bone implant, such as a cranium (skull) implant, has an elasticity modulus of 50-120 GPa, as measured by nanoindentation.

In still another embodiment of the present invention, the bone implant, such as a cranium (skull) implant, has an elasticity modulus of 60-110 GPa, as measured by nanoindentation.

In still another embodiment of the present invention, the bone implant, such as a cranium (skull) implant, has an elasticity modulus of 70-100 GPa, as measured by nanoindentation.

In still another embodiment of the present invention, the bone implant, such as a cranium (skull) implant, has an elasticity modulus of 80-90 GPa, as measured by nanoindentation.

In one embodiment of the present invention, the entire surface of the tantalum substrate is covered with a porous lithium tantalate membrane.

In one embodiment of the present invention, the entire surface of the niobium substrate is covered with a porous lithium niobate membrane.

In one embodiment of the present invention, the entire surface of the tantalum-niobium alloy substrate is covered with a porous lithium tantalate-lithium niobate mixture membrane.

In one embodiment of the present invention, the entire surface of the titanium substrate is covered with a porous titanium oxide membrane.

From a biomechanical point of view, the elasticity modulus of a dental implant has an effect on stress distribution at a bone interface. Generally, the higher the elasticity modulus of a dental implant, the smaller the internal stress of the bone around the neck and the greater the internal stress of the bone at the root; the lower the elasticity modulus of a dental implant, the greater the relative displacement motion of the dental implant and the bone interface. When the elasticity modulus of a dental implant is similar to that of cortical bone, cancellous bone, the biomechanical compatibility is relatively poor. A suitable elasticity modulus of a dental implant is 70 GPa or more. The bone implants of the present invention, including dental implants, have an elasticity modulus of at least 150 GPa. Therefore, the dental implant of the present invention has a very suitable elasticity modulus and an excellent biomechanical compatibility.

In one embodiment of the present invention, examples of the bone implant include bone prostheses, such as cranium and dental implants.

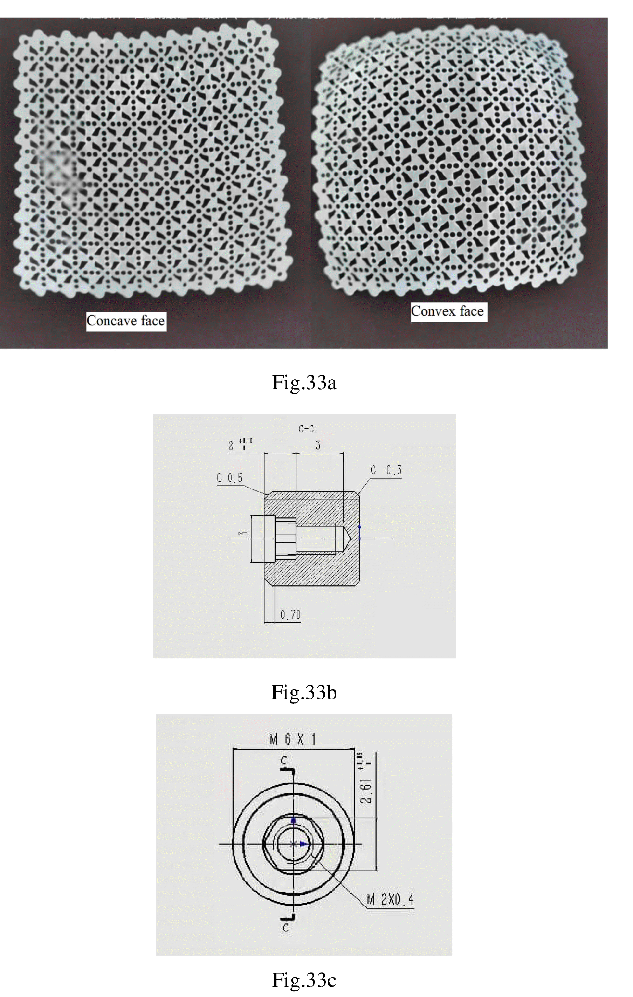

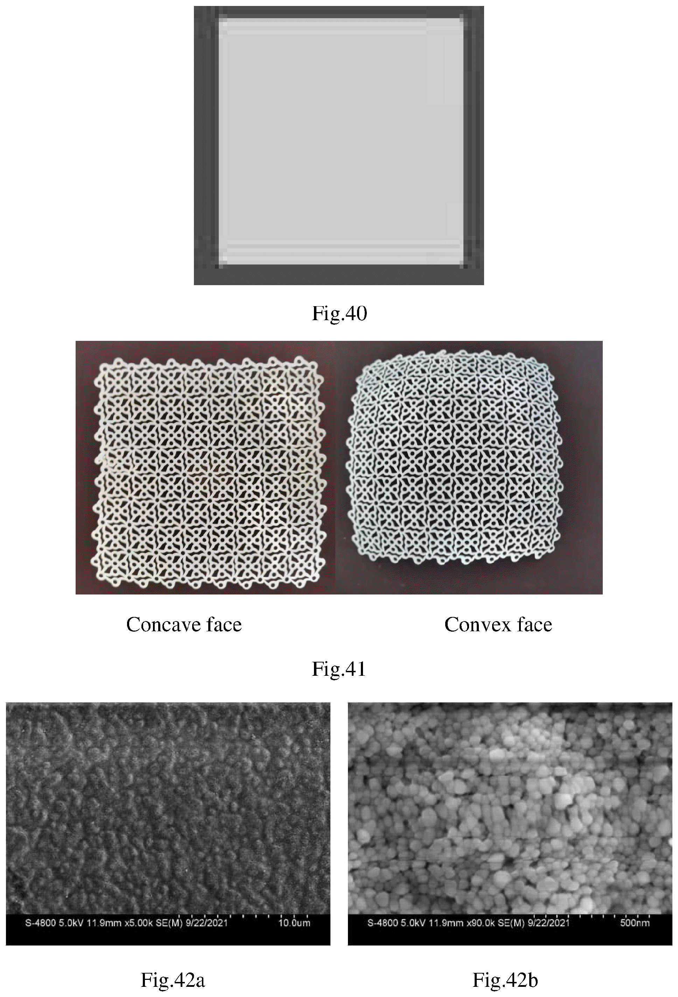

In one embodiment of the present invention, the cranium is porous, such as the tantalum cranium shown in Figure 33a and the niobium cranium shown in Figure 41.

In one embodiment of the present invention, the dental implant comprises a denture abutment, a main body and a neck connecting the denture abutment and the main body. The method for the preparation of the dental implant is known to those skilled in the art, and reference can be made to, for example, CN109758245A, CN109965996A and CN110610046A.

In a preferred embodiment of the present invention, the dental implant comprises a denture abutment, a main body and a neck connecting the denture abutment and the main body, wherein the main body is provided with external threads in contact with the alveolar bone on the outer surface thereof.

In one embodiment of the present invention, the bone implant has a similar color to the bone. In a preferred embodiment of the present invention, the bone implant is white, particularly for the denture abutment.

In one embodiment of the present invention, the substrate is porous. The porous substrate has excellent mechanical properties and tissue compatibility. Human tissue reconstruction, bone grafting, replacement and the like can all be realized by implanting a porous material into a bone-deficient part; the bone grows into a host interface along porous pores so that the porous material is bound fully and tightly and integrated with a bone tissue. After being implanted into a human body as a support material, the porous material does not need to degrade in the human body due to its excellent tissue compatibility, so there is no need to remove it by a secondary surgery. A porous metal is solid in texture and superior to a spongy bone, a ceramic product and a freeze-dried bone chip in respect of both wear resistance and fatigue resistance, and can provide sufficient physiological load.

Tantalum, niobium, tantalum-niobium alloys and titanium are refractory metals, and the abovementioned porous metals are generally prepared by the methods for the preparation of refractory metals. For example, porous tantalum can be prepared either by powder sintering by a known method or by a vapor deposition method. Other methods for preparing porous tantalum in the art can also be used.

In one embodiment of the present invention, the tantalum used is dense. Prior art documents all use porous tantalum as a medical orthopedic material and are silent about the use of a dense tantalum metal as a medical orthopedic material. With regard to dental implants, from a biomechanical point of view, the elasticity modulus of a dental implant has an effect on stress distribution at a bone interface. Generally, the higher the elasticity modulus of a dental implant, the smaller the internal stress of the bone around the neck and the greater the internal stress of the bone at the root; the lower the elasticity modulus of a dental implant, the greater the relative displacement motion of the dental implant and the bone interface. When the elasticity modulus of a dental implant is similar to that of cortical bone, cancellous bone, the biomechanical compatibility is relatively poor. A suitable elasticity modulus of a dental implant is 70 GPa or more. Since a dental implant often assumes the function of chewing and carries a very heavy load, the tensile strength and elasticity modulus of porous tantalum cannot meet the load requirement. The dense metal tantalum is relatively high in terms of tensile strength and elasticity modulus and is more suitable for use in a dental implant.

One embodiment of the present invention is to coat a porous tantalum dental implant with a layer of porous lithium tantalate membrane, thereby improving the mechanical properties, such as tensile strength and elasticity modulus, of the dental implant.

One embodiment of the present invention is to coat a porous niobium dental implant with a layer of porous lithium niobate membrane, thereby improving the mechanical properties, such as tensile strength and elasticity modulus, of the dental implant.

One embodiment of the present invention is to coat a porous tantalum niobium dental implant with a layer of porous lithium tantalate-lithium niobate mixture membrane, thereby improving the mechanical properties, such as tensile strength and elasticity modulus, of the dental implant.

Another embodiment of the present invention is to coat dense metal tantalum with a layer of porous lithium tantalate membrane to allow its tensile strength and elasticity modulus to become higher, which makes it possible to better meet the requirements for the tensile strength and elasticity modulus of the dental implant. Moreover, the tantalum substrate has a porous lithium tantalate membrane on its surface so that the dental implant can be better compatible with human biological tissues, which makes it possible that human biological tissues can be bound with the tantalum substrate through the porous lithium tantalate membrane, and makes it possible that the tantalum material can be bound fully and more tightly and integrated with human tissues, such as alveolar bone.

In one embodiment of the present invention, it is possible that a non-exposed part of a bone implant, such as a dental implant, is not coated with a layer of lithium tantalate membrane, but is made using only a pure tantalum material. The denture abutment and the neck are preferably coated with a lithium tantalate membrane to increase aesthetic appearance.

The methods for the preparation of a dense tantalum material are known to those skilled in the art, e.g., it can be obtained by such methods as casting and rolling.

In one embodiment of the present invention, the method for preparing the bone implant comprises: (1) providing a tantalum substrate; and (2) forming a layer of porous lithium tantalate membrane on the tantalum substrate.

In one embodiment of the present invention, the method for preparing the bone implant comprises: (1) providing a niobium substrate; and (2) forming a layer of porous lithium niobate membrane on the niobium substrate.

In one embodiment of the present invention, the method for preparing the bone implant comprises: (1) providing a tantalum-niobium alloy substrate; and (2) forming a layer of porous lithium tantalate-lithium niobate mixture membrane on the tantalum-niobium alloy substrate.

In one embodiment of the present invention, the method for preparing the bone implant comprises: (1) providing a titanium substrate; and (2) forming a layer of porous titanium oxide membrane on the titanium substrate.

In one embodiment of the present invention, the substrate has a shape desired for the bone implant, e.g., the shapes of a dental implant and a cranium.

In a preferred embodiment of the present invention, a tantalum substrate having a desired bone implant shape is coated with a layer of porous lithium tantalate membrane.

In another embodiment of the present invention, a tantalum substrate having a desired bone implant shape is coated with a layer of porous lithium tantalate membrane; moreover, the unwanted porous lithium tantalate membrane on the bone implant is removed by cutting or grinding, and the porous lithium tantalate membrane is retained only on a portion of the surface of the bone implant. Concretely speaking, a plurality of unions manufactured, which are qualified in appearance and size and whose inner pores have not been processed, are coated with a lithium tantalate membrane, and then the inner pores are processed; in this way, the inner pores can be kept uncoated.

In a preferred embodiment of the present invention, a tantalum substrate having a desired bone implant shape is coated with a layer of porous lithium tantalate membrane on a portion of the surface thereof.

In one embodiment of the present invention, the porous membrane has a thickness of 1-20 µm, preferably 2-10 µm, more preferably 3-5 µm.

In step (2), the porous lithium tantalate membrane is formed by a molten salt electrochemical method.

In one embodiment of the present invention, in step (2), a tantalum substrate is placed in an oxygen-containing inorganic lithium salt (such as LiNO₃) or a mixed melt of an oxygen-containing inorganic lithium salt and lithium hydroxide, or a mixed molten liquid of a salt and lithium hydroxide or a mixed molten liquid of a lithium salt and an oxygen-containing salt, at a temperature of 250°C to 650°C, and an anode voltage of 1 to 66V is applied, which voltage is maintained for 0.01 to 200 hours, with a boosting current density of 1 to 1000 mA/cm², to form a porous lithium tantalate membrane.

In another embodiment of the present invention, in step (2), a tantalum substrate is placed in an oxygen-containing inorganic lithium salt (such as LiNO₃) or a mixed melt of an oxygen-containing inorganic lithium salt and lithium hydroxide, or a mixed molten liquid of a salt and lithium hydroxide or a mixed molten liquid of a lithium salt and an oxygen-containing salt, at a temperature of 440°C to 600°C, and an anode voltage of 10 to 30V is applied, which voltage is maintained for 5 minutes to 10 hours, with a boosting current density of 1 to 1000 mA/cm², to form a porous lithium tantalate membrane.

In yet another embodiment of the present invention, in step (2), a tantalum substrate is placed in an oxygen-containing inorganic lithium salt (such as LiNO₃) or a mixed melt of an oxygen-containing inorganic lithium salt and lithium hydroxide, or a mixed molten liquid of a salt and lithium hydroxide or a mixed molten liquid of a lithium salt and an oxygen-containing salt, at a temperature of 570°C to 598°C, and an anode voltage of 10 to 20V is applied, which voltage is maintained for 8 to 30 minutes, with a boosting current density of 5 to 20 mA/cm², to form a porous lithium tantalate membrane.

In a preferred embodiment, in step (2), an ultrasonic generator can be placed in the mixed melt or the mixed molten liquid.

Preferably, the molten salt electrochemical method in step (2) is a molten lithium salt electrochemical method.

Before step (2), the step of anodizing a tantalum substrate can be carried out.

In the anodizing step, a Ta₂O₅ membrane, such as an amorphous Ta₂O₅ membrane, is formed on the tantalum substrate. Those without pores are suitable for use on tantalum capacitors.

A Ta₂O₅ membrane may also be porous, which is more advantageous for the preparation of a porous lithium tantalate membrane. Specifically, an implant is placed in concentrated sulfuric acid at a temperature of 190°C to 245°C (i.e., a porous Ta₂O₅ membrane is formed in 98% concentrated sulfuric acid).

In one embodiment of the present invention, a layer of amorphous Ta₂O₅ membrane is formed on a tantalum substrate by anodic oxidation in the anodizing step. Particularly, in the anodizing step, a tantalum substrate is placed in an oxygen-containing electrolyte solution at a temperature of room temperature to 380°C, preferably room temperature to 300°C, and an anode voltage of 3 to 800V is applied, which voltage is maintained for 0.01 to 2 hours, with a boosting current density of 1 to 200 mA/cm², to form a layer of amorphous Ta₂O₅ membrane.

In the anodizing step, if the temperature of the solution is high, the voltage applied should be low; otherwise, it can be high. For example, for a 0.01% H₃PO₄ solution at room temperature, a maximum voltage of 600V can be applied. The voltage applied should be below the flash voltage of the solution regardless of the type of the solution.

In the anodizing step, the oxygen-containing electrolyte solution may be an aqueous solution, a non-aqueous solution, or a mixture of an aqueous electrolyte and an organic compound.

An aqueous oxygen-containing electrolyte may be, for example, an aqueous acid, alkali, salt. The solution temperature is from room temperature (about 25°C) to 95°C, the anode voltage is from 5 to 600V, and the voltage is maintained for 60 to 90 minutes. If the temperature is too high, the moisture will evaporate too fast. When the solution temperature is relatively high, the anode voltage should be relatively low.

A non-aqueous oxygen-containing electrolyte may be an anhydrous concentrated sulfuric acid or a molten salt or a mixture of a molten salt and an alkali, such as potassium nitrate, sodium nitrate, lithium nitrate, or their mixtures with alkali of lithium, sodium, potassium, etc.

Another type of oxygen-containing electrolyte solution may be a mixture of an aqueous electrolyte and an organic compound, such as ethanol, ethylene glycol and n-butanol. The temperature of the aqueous solution should be 95°C or lower, otherwise the water volatilizes rapidly and is difficult to control.

In one embodiment of the present invention, the preparation method of the present invention comprises nitriding or carburizing a tantalum substrate in advance. However, nitriding or carburizing treatment may not be performed either. In order to obtain a relatively high hardness, nitriding or carburizing treatment can be performed.

In a preferred embodiment, an ion nitriding furnace, which is filled with nitrogen and hydrogen in a nitrogen:hydrogen ratio of 2:1 to 1:10, is used with a tantalum substrate as a cathode under the conditions of a furnace temperature of 500 to 1000°C and a furnace pressure of 20 to 2000Pa, for nitriding for 0.5 to 6 hours, so that a nitrided layer is formed on the surface of the tantalum substrate and the hardness of the tantalum substrate after nitriding is controlled between HV180 to 480.

According to one embodiment of the present invention, there is provided a method for preparing a dental implant, comprising:
1) selecting a tantalum rod, or a niobium rod, a tantalum-niobium alloy rod, or a titanium rod with an appropriate diameter;
2) providing a processed cylindrical or conical implant (including processing external threads and internal pore threads, etc., and providing a connector and an abutment that match the implant);
3) performing an electrochemical coating treatment in lithium-containing salt melt,
wherein a white lithium tantalate membrane, a lithium niobate membrane, or a lithium tantalate-lithium niobate mixture membrane may be formed on the tantalum, niobium, or tantalum-niobium alloy implant, and a layer of porous titanium oxide membrane is formed on the surface of the titanium implant.

The present invention proposes to replace threaded ribs with suitable pores. The pores can go deep into the inner wall of the implant, i.e., perforations, without affecting the strength of the implant. The advantage of this solution is that it can increase the contact surface between the implant and the human bone and increase the compatibility and firmness of the implant.

Of course, the outer pores may not be perforated and a certain thickness is reserved, so that the inner pores of the implant can be threaded, which facilitates the immobilization of the connector or abutment.

According to one embodiment of the present invention, there is provided a method for preparing a dental implant, comprising:
1) providing a dental implant blank and designing punching parameters,
   i.e., determining the size of the cylindrical or conical implant, including the outer diameter of the cylinder and the diameter of the inner pores, expanding the cylinder with pores into a planar view, and designing and determining the size of the pores, the pore spacing or the pore distribution, as well as the punching depth of the pores and the shape of the pores, i.e., a straight pore or a tapered pore;
2) punching pores,
   i.e., punching pores according to design requirements through a method for punching a spinneret plate or a laser;
3) polishing,
   i.e., after punching, the excess material on the surface should be removed by polishing, e.g., upon punching with a laser, there will generally be no excess material around the pores, and polishing is relatively easy;
4) welding the punched and polished tantalum flake with argon arc welding or laser welding into the originally designed cylindrical or conical implant, and performing necessary repairs and processing internal threads, etc.;
5) performing the surface treatment on the porous implant and the abutment by an electrochemical method to form a complex porous membrane corresponding to the implant metal and obtain a coated dental implant with pores.

According to one embodiment of the present invention, there is provided a method for perforating an implant, comprising:
1) a perforation inlet, i.e., a pore on the side that is intended to come into contact with a human bone, which has a diameter controlled within the range of 0.08 to 0.30 mm, preferably 0.16 to 0.22 mm;
2) an outlet, i.e., a pore at the end where the pore meets the inner pore of the implant, which has a diameter controlled within the range of 0.28 to 0.06 mm, preferably 0.22 to 0.13 mm, and may be a tapered pore or a straight pore, wherein the tapered pore may be more beneficial to guiding the growth of the human bone into the implant.

According to one embodiment of the present invention, a design of a non-perforated implant is provided to ensure the processing of threads and the like to be processed for the installation of an abutment connector, on the inner wall of the central pores of the dental implant; the pores on the implant should be 0.6 to 0.8 mm, otherwise it will not be perforated so that the inner pores are threaded.

In the context of the present invention, niobium and tantalum-niobium alloy substrates are also applicable to embodiments concerning a tantalum substrate.

Unless otherwise specified, tensile strength and elasticity modulus are tested according to the GB/T22315-2008 standard.

The specific embodiments described below are helpful for those skilled in the art to understand the present invention, but are not used to limit the scope of the present invention.
1. A bone implant comprising:
   (1) a substrate; and
   (2) a porous membrane on the substrate,

   wherein the substrate is selected from the group consisting of a tantalum substrate, a niobium substrate, a tantalum-niobium alloy substrate and a titanium substrate, preferably the substrate is selected from the group consisting of a tantalum substrate, a niobium substrate and a tantalum-niobium alloy substrate, and
   wherein the porous membrane is selected from the group consisting of a porous lithium tantalate membrane, a porous lithium niobate membrane, a porous lithium tantalate-lithium niobate mixture membrane and a porous titanium oxide membrane, preferably the porous membrane is selected from the group consisting of a porous lithium tantalate membrane, a porous lithium niobate membrane and a porous lithium tantalate-lithium niobate mixture membrane.
2. The bone implant according to Embodiment 1, comprising:
   (1) a tantalum substrate, a niobium substrate, a tantalum-niobium alloy substrate or a titanium substrate; and
   (2) a porous lithium tantalate membrane on the tantalum substrate, wherein the lithium tantalate membrane contains a tantalum oxide,
      a porous lithium niobate membrane on the niobium substrate, wherein the lithium niobate membrane contains a niobium oxide,
      a porous lithium tantalate-lithium niobate mixture membrane on the tantalum-niobium alloy substrate, wherein the mixture membrane contains a tantalum oxide and a niobium oxide, or
      a porous titanium oxide membrane on the titanium substrate.
3. The bone implant according to Embodiment 1 or 2, wherein the porous lithium tantalate membrane, the porous lithium niobate membrane, the porous lithium tantalate-lithium niobate mixture membrane or the porous titanium oxide membrane has a pore diameter of 0.1-1 µm.
4. The bone implant according to Embodiment 1 or 2, wherein the porous lithium tantalate membrane, the porous lithium niobate membrane, the porous lithium tantalate-lithium niobate mixture membrane or the porous titanium oxide membrane has a thickness of 1-20 µm, preferably 2-10 µm, more preferably 3-5 µm.
5. The bone implant according to Embodiment 1 or 2, wherein the bone implant has an elasticity modulus of at least 150 GPa.
6. The bone implant according to Embodiment 1 or 2, wherein the bone implant has an elasticity modulus of at least 170 GPa.
7. The bone implant according to Embodiment 1 or 2, wherein the bone implant has an elasticity modulus of 150-200 GPa.
8. The bone implant according to Embodiment 7, wherein the bone implant has an elasticity modulus of 170-190 GPa.
9. The bone implant according to Embodiment 1 or 2, wherein the bone implant has a similar color to a bone.
10. The bone implant according to Embodiment 1 or 2, wherein the bone implant comprises a bone prosthesis, a dental implant and a cranium.
11. The bone implant according to Embodiment 10, wherein the dental implant comprises a denture abutment, a main body and a neck connecting the denture abutment and the main body.
12. The bone implant according to Embodiment 1 or 2, wherein the bone implant has an elasticity modulus of 10-160 GPa, preferably 20-150 GPa, more preferably 30-140 GPa.
13. The bone implant according to Embodiment 1 or 2, wherein the tantalum substrate, the niobium substrate or the tantalum-niobium alloy substrate is porous.
14. The bone implant according to Embodiment 1 or 2, wherein the tantalum substrate, the niobium substrate or the tantalum-niobium alloy substrate is dense.
15. A method for preparing a bone implant according to any one of Embodiments 1-14, comprising:
   (1) providing a substrate; and
   (2) forming a porous membrane on the substrate,
      wherein the substrate is selected from the group consisting of a tantalum substrate, a niobium substrate, a tantalum-niobium alloy substrate and a titanium substrate, preferably the substrate is selected from the group consisting of a tantalum substrate, a niobium substrate and a tantalum-niobium alloy substrate, and
      wherein the porous membrane is selected from the group consisting of a porous lithium tantalate membrane, a porous lithium niobate membrane, a porous lithium tantalate-lithium niobate mixture membrane and a porous titanium oxide membrane, preferably the porous membrane is selected from the group consisting of a porous lithium tantalate membrane, a porous lithium niobate membrane and a porous lithium tantalate-lithium niobate mixture membrane.
16. The method according to Embodiment 15, comprising:
   (1) providing a tantalum substrate, a niobium substrate, a tantalum-niobium alloy substrate or a titanium substrate; and
   (2) forming a porous lithium tantalate membrane, a porous lithium niobate membrane, or a porous lithium tantalate-lithium niobate mixture membrane on the tantalum substrate, niobium substrate or tantalum-niobium alloy substrate, or forming a porous titanium oxide membrane on the titanium substrate,

   wherein the lithium tantalate membrane contains a tantalum oxide,
   wherein the lithium niobate membrane contains a niobium oxide, and
   wherein the mixture membrane contains a tantalum oxide and a niobium oxide.
17. The method according to Embodiment 15 or 16, wherein in step (2), a porous membrane is formed by a molten salt electrochemical method.
18. The method according to Embodiment 15 or 16, wherein a Ta₂O₅ membrane is formed on a tantalum substrate prior to step (2).
19. The method according to Embodiment 18, wherein an amorphous Ta₂O₅ membrane is formed on a tantalum substrate by anodic oxidation.
20. The method according to Embodiment 19, wherein in the step of forming a Ta₂O₅ membrane on a tantalum substrate, a tantalum substrate is placed in an oxygen-containing electrolyte solution at a temperature of room temperature to 380°C, preferably room temperature to 300°C, and an anode voltage of 3 to 800V is applied, which voltage is maintained for 0.01 to 2 hours, with a boosting current density of 1 to 200 mA/cm², to form an amorphous Ta₂O₅ membrane.
21. The method according to Embodiment 15 or 16, wherein an Nb₂O₅ membrane is formed on a niobium substrate prior to step (2).
22. The method according to Embodiment 21, wherein an amorphous Nb₂O₅ membrane is formed on a niobium substrate by anodic oxidation.
23. The method according to Embodiment 22, wherein in the step of forming an Nb₂O₅ membrane on a niobium substrate, a niobium substrate is placed in an oxygen-containing electrolyte solution at a temperature of room temperature to 380°C, preferably room temperature to 300°C, and an anode voltage of 3 to 800V is applied, which voltage is maintained for 0.01 to 2 hours, with a boosting current density of 1 to 200 mA/cm², to form an amorphous Nb₂O₅ membrane.
24. The method according to Embodiment 15 or 16, wherein a Ta₂O₅-Nb₂O₅ mixture membrane is formed on a tantalum-niobium alloy substrate prior to step (2).
25. The method according to Embodiment 24, wherein an amorphous Ta₂O₅-Nb₂O₅ mixture membrane is formed on a tantalum-niobium alloy substrate by anodic oxidation.
26. The method according to Embodiment 25, wherein in the step of forming a Ta₂O₅-Nb₂O₅ mixture membrane on a tantalum-niobium alloy substrate, a tantalum-niobium alloy substrate is placed in an oxygen-containing electrolyte solution at a temperature of room temperature to 380°C, preferably room temperature to 300°C, and an anode voltage of 3 to 800V is applied, which voltage is maintained for 0.01 to 2 hours, with a boosting current density of 1 to 200 mA/cm², to form an amorphous Ta₂O₅-Nb₂O₅ mixture membrane.
27. The method according to any one of Embodiments 20, 23 and 26, wherein the oxygen-containing electrolyte solution is an aqueous solution, a non-aqueous solution, or a mixture of an aqueous electrolyte and an organic compound.
28. The method according to Embodiment 27, wherein the molten salt electrochemical method is a molten lithium salt electrochemical method.
29. The method according to Embodiment 15 or 16, wherein a tantalum substrate, a niobium substrate or a tantalum-niobium alloy substrate is placed in an oxygen-containing inorganic lithium salt or a mixed melt of an oxygen-containing inorganic lithium salt and lithium hydroxide, or a mixed molten liquid of a salt and lithium hydroxide or a mixed molten liquid of a lithium salt and an oxygen-containing salt, at a temperature of 250°C to 650°C, and an anode voltage of 1 to 66V is applied, which voltage is maintained for 0.01 to 200 hours, with a boosting current density of 1 to 1000 mA/cm², to form a membrane layer containing a lithium-containing compound.
30. The method according to Embodiment 29, wherein in step (2), a tantalum substrate, a niobium substrate or a tantalum-niobium alloy substrate is placed in an oxygen-containing inorganic lithium salt or a mixed melt of an oxygen-containing inorganic lithium salt and lithium hydroxide, or a mixed molten liquid of a salt and lithium hydroxide or a mixed molten liquid of a lithium salt and an oxygen-containing salt, at a temperature of 440°C to 600°C, and an anode voltage of 10 to 30V is applied, which voltage is maintained for 5 minutes to 10 hours, with a boosting current density of 5 to 20 mA/cm², to form a membrane layer containing a lithium-containing compound.
31. The method according to Embodiment 29, wherein in step (2), a tantalum substrate, a niobium substrate or a tantalum-niobium alloy substrate is placed in an oxygen-containing inorganic lithium salt or a mixed melt of an oxygen-containing inorganic lithium salt and lithium hydroxide, or a mixed molten liquid of a salt and lithium hydroxide or a mixed molten liquid of a lithium salt and an oxygen-containing salt, at a temperature of 570°C to 598°C, and an anode voltage of 10 to 20V is applied, which voltage is maintained for 8 to 30 minutes, with a boosting current density of 5 to 20 mA/cm², to form a membrane layer containing a lithium-containing compound.
32. The method according to Embodiment 29, wherein the oxygen-containing inorganic lithium salt is LiNO₃.
33. The method according to Embodiment 29, wherein in step (c), an ultrasonic generator is placed in the mixed melt or the mixed molten liquid.
34. The method according to Embodiment 15 or 16, wherein a tantalum substrate, a niobium substrate or a tantalum-niobium alloy substrate is nitrided or carburized.
35. A use of a bone implant in a medical material, wherein the bone implant is as defined in any one of Embodiments 1-14 or is prepared according to the method of any one of Embodiments 15-34.
36. The use according to Embodiment 35, wherein the medical material is a dental implant or a cranium.

### Examples

The nature and purpose of the present invention will be further understood by examples in conjunction with figures below. It should be understood that these examples are for illustrative purpose only and are not intended to limit the scope of the present invention.

### Example 1

Step 1: The metal tantalum was processed into a dense tantalum flake.

Step 2: An anode voltage of 10V was applied for the tantalum flake in a molten lithium nitrate solution at 570°C, and reaction was carried out at said voltage for 30 minutes, to form a porous lithium tantalate membrane layer on the tantalum flake.

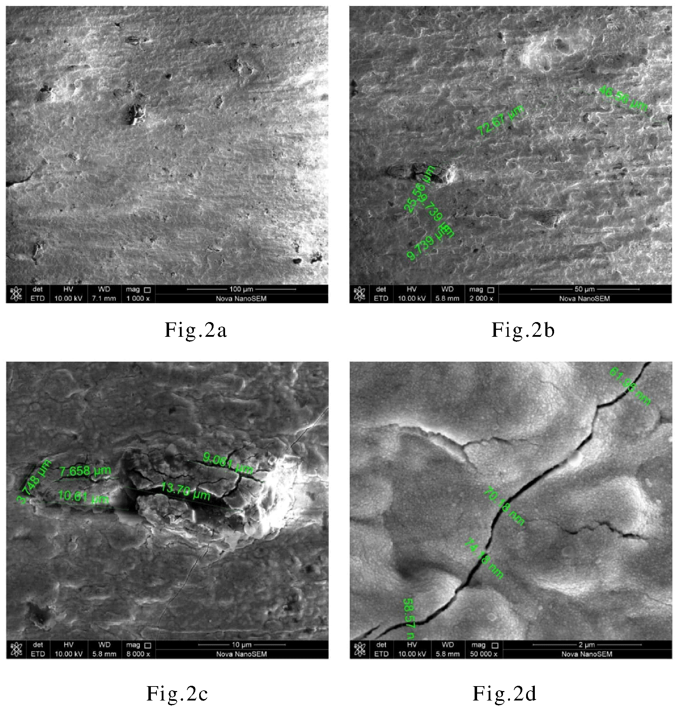

Figures 2a-2d show SEM photographs of the sample of Example 1.

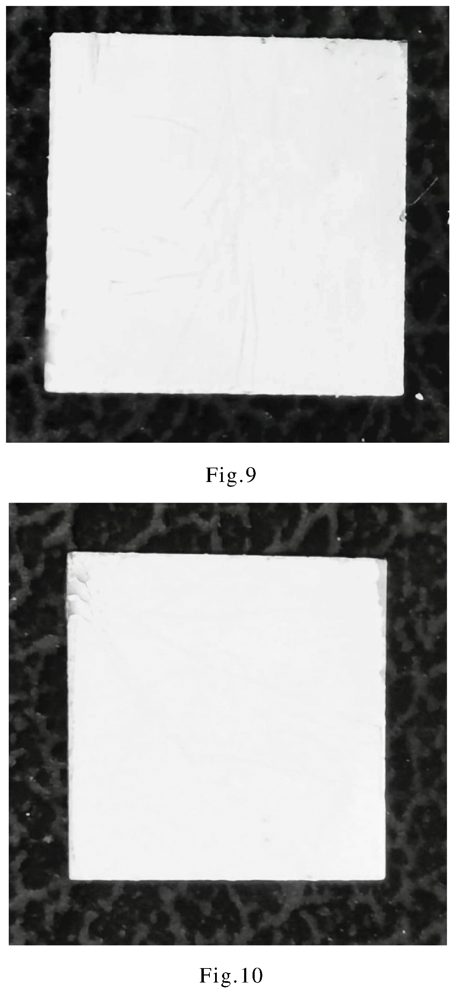

Figure 9 shows an appearance photograph of the sample of Example 1.

### Example 2

Step 1: The metal tantalum was processed into a dense tantalum flake.

Step 2: An anode voltage of 10V was applied for the tantalum flake in a molten lithium nitrate solution at 580°C, and reaction was carried out at said voltage for 25 minutes, to form a porous lithium tantalate membrane layer on the tantalum flake.

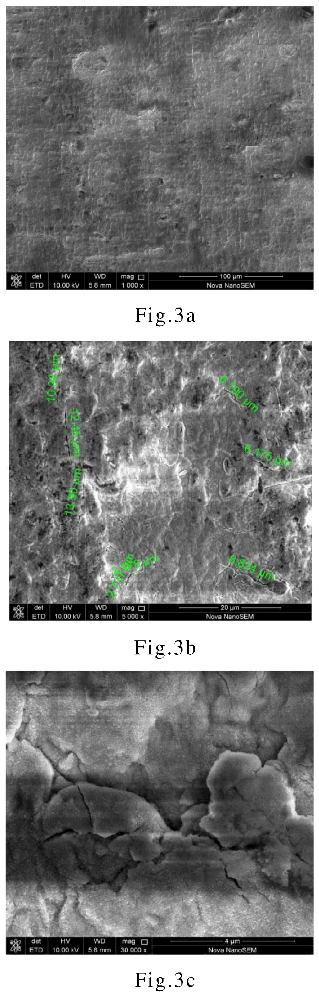

Figures 3a-3c show SEM photographs of the sample of Example 2.

Figure 10 shows an appearance photograph of the sample of Example 2.

### Example 3

Step 1: The metal tantalum was processed into a dense tantalum flake.

Step 2: An anode voltage of 10V was applied for the tantalum flake in a molten lithium nitrate solution at 598°C, and reaction was carried out at said voltage for 10 minutes, to form a porous lithium tantalate membrane layer on the tantalum flake.

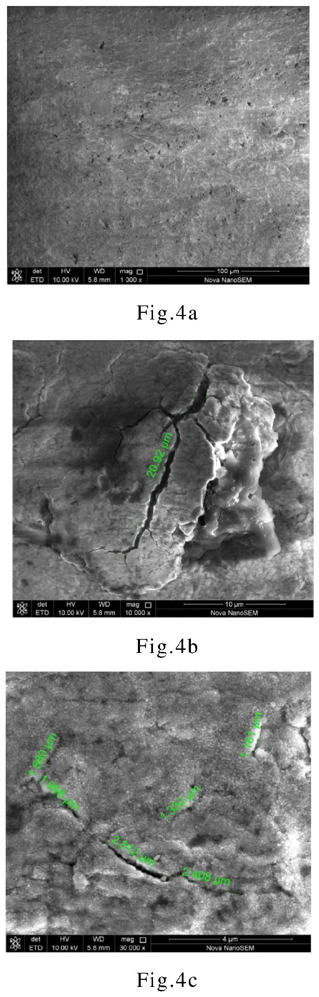

Figures 4a-4c show SEM photographs of the sample of Example 3.

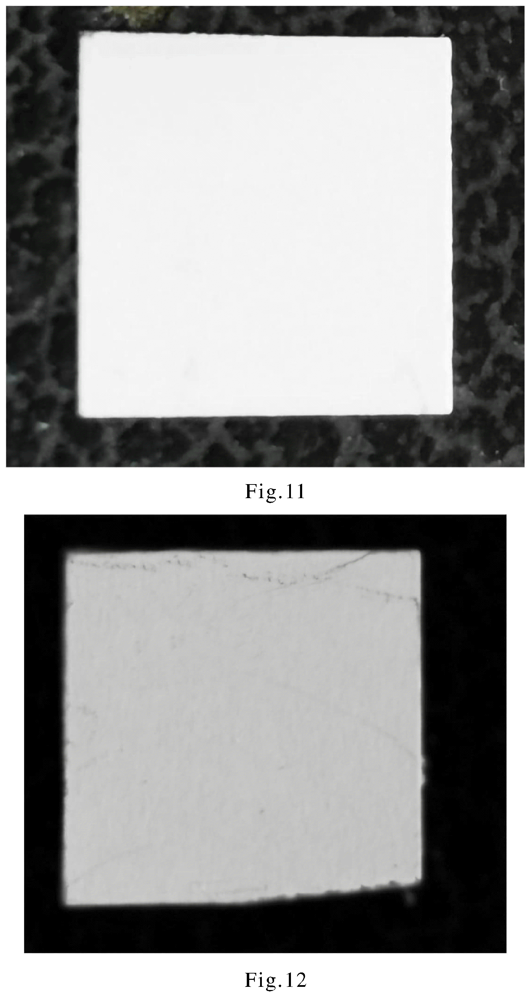

Figure 11 shows an appearance photograph of the sample of Example 3.

### Example 4

Step 1: The metal tantalum was processed into a dense tantalum flake.

Step 2: An anode voltage of 60V was applied for the tantalum flake in a solution having a volume ratio of ethylene glycol:0.01% H₃PO₄ of 2:1 and a temperature of 90°C, and said voltage was held for 3 hours, followed by cleaning.

Step 3: An anode voltage of 10V was applied for the tantalum flake obtained in step (2) in a molten lithium nitrate solution at 598°C, and reaction was carried out at said voltage for 8 minutes, to form a porous lithium tantalate membrane layer on the tantalum flake.

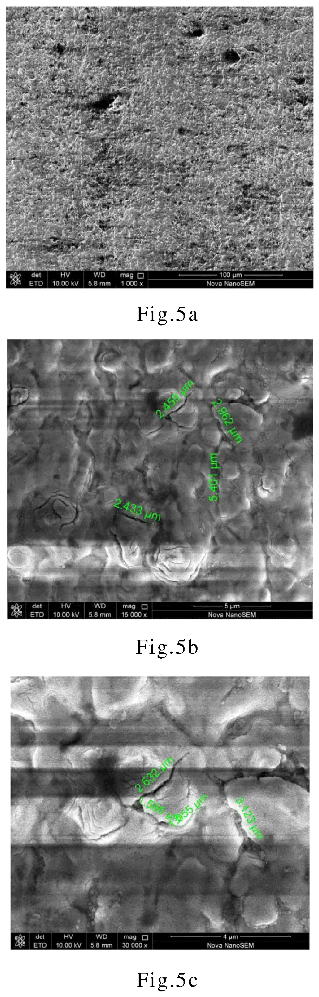

Figures 5a-5c show SEM photographs of the sample of Example 4.

Figure 12 shows an appearance photograph of the sample of Example 4.

### Example 5

Step 1: The metal tantalum was processed into a dense tantalum flake.

Step 2: An anode voltage of 143V was applied for the tantalum flake in a solution having a volume ratio of ethylene glycol:0.01% H₃PO₄ of 2:1 and a temperature of 90°C, and said voltage was held for 3 hours, followed by cleaning.

Step 3: An anode voltage of 28V was applied for the tantalum flake obtained in step (2) in a molten lithium nitrate solution at 482°C, and reaction was carried out at said voltage for 6.5 hours, to form a porous lithium tantalate membrane layer on the tantalum flake.

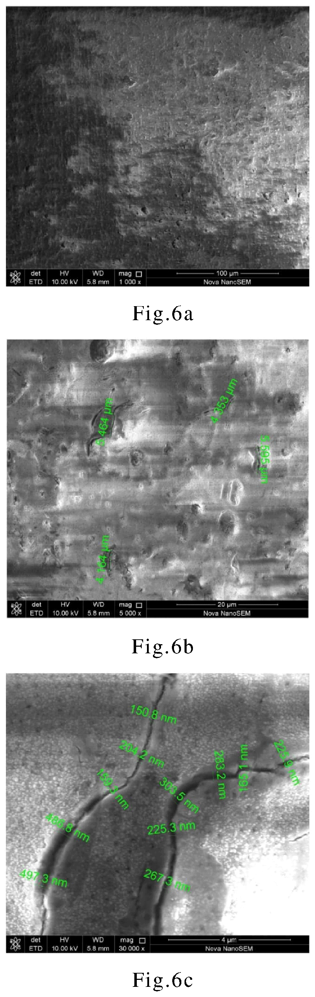

Figures 6a-6c show SEM photographs of the sample of Example 5.

### Example 6

Step 1: The metal tantalum was processed into a dense tantalum flake.

Step 2: An anode voltage of 33V was applied for the tantalum flake in a molten lithium nitrate solution at 440°C, and reaction was carried out at said voltage for 12 hours, to form a porous lithium tantalate membrane layer on the tantalum flake.

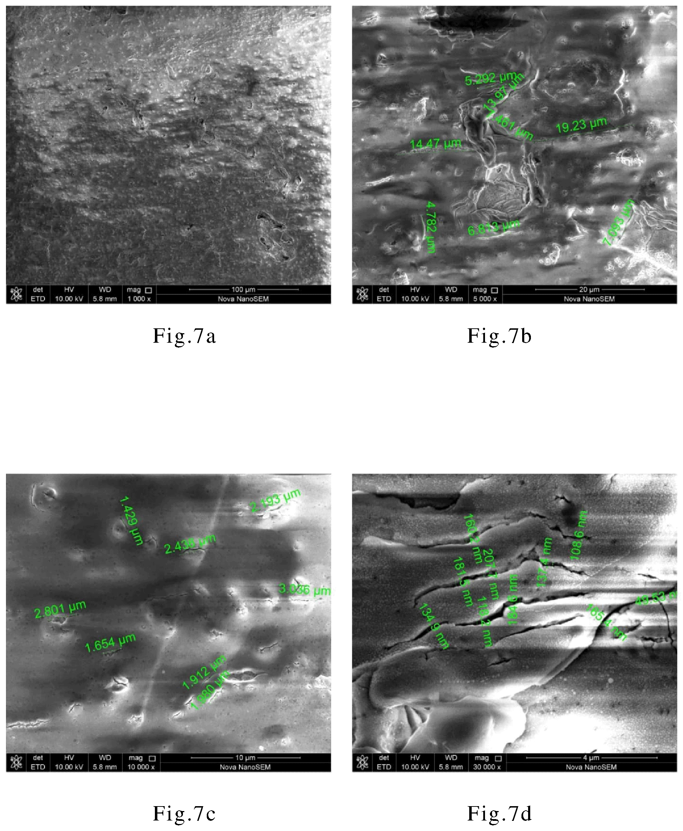

Figures 7a-7d show SEM photographs of the sample of Example 6.

### Example 7

Step 1: The metal tantalum was processed into a dense tantalum flake.

Step 2: An anode voltage of 60V was applied for the tantalum flake in a solution having a volume ratio of ethylene glycol:0.01% H₃PO₄ of 2:1 and a temperature of 90°C, and said voltage was held for 3 hours, followed by cleaning.

Step 3: An anode voltage of 10V was applied for the tantalum flake obtained in step (2) in a molten lithium nitrate solution at 598°C, and reaction was carried out at said voltage for 10 minutes, to form a porous lithium tantalate membrane layer on the tantalum flake.

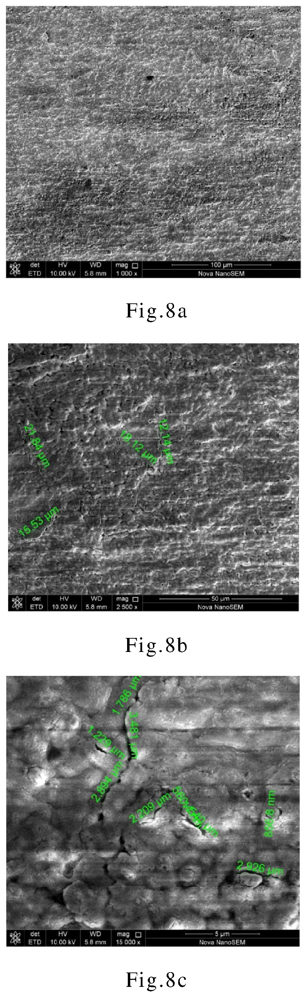

Figures 8a-8c show SEM photographs of the sample of Example 7.

### Example 8

Step 1: The metal tantalum was processed into a dense tantalum flake and a tantalum dental implant, respectively.

Step 2: An anode voltage of 10V was applied for the tantalum flake and the tantalum dental implant (a threaded tantalum rod having a diameter of 6 mm, with pores having a diameter of 2 mm), respectively, in a molten lithium nitrate:potassium nitrate (in a weight ratio of 1:1) solution at 580°C, and reaction was carried out at said voltage for 30 minutes, to form a porous lithium tantalate membrane layer on the tantalum flake and the tantalum dental implant.

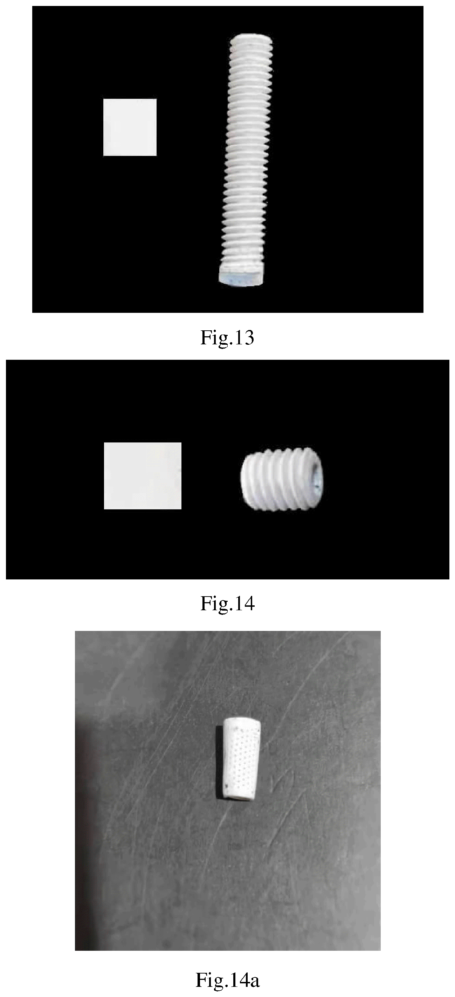

Figure 13 shows an appearance photograph of the sample of Example 8.

### Example 9

Step 1: The metal tantalum was processed into a dense tantalum flake and a tantalum dental implant (a threaded tantalum rod having a diameter of 6 mm, with pores having a diameter of 2 mm), respectively.

Step 2: First of all, an anode voltage of 60V was applied for the tantalum flake and the tantalum dental implant, respectively, in 0.05 wt% of a H₃PO₄ aqueous solution at 90°C, and said voltage was held for 1.5 hours, followed by cleaning.

Step 3: An anode voltage of 10V was applied for the tantalum flake and the tantalum dental implant obtained in step (2) in molten lithium nitrate:potassium nitrate (in a weight ratio of 1:1) at 598°C, and reaction was carried out at said voltage for 8 minutes, to form a porous lithium tantalate membrane layer on the tantalum flake and the tantalum dental implant.

Figure 14 shows an appearance photograph of the sample of Example 9.

### Example 9a

An anode voltage of 10V was applied across a porous tantalum dental implant in molten lithium nitrate:potassium nitrate (in a weight ratio of 1:1) at 580°C, and reaction was carried out at said voltage for 25 minutes.

Figure 14a shows an appearance photograph of the sample of Example 9a.

### Comparative Example 1

The metal tantalum was processed into a dense tantalum flake without the step of forming a porous lithium tantalate membrane layer on the tantalum flake.

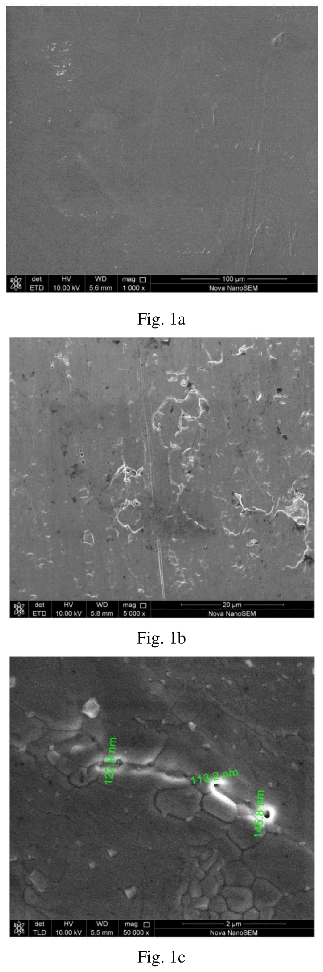

Figures 1a-1c show SEM photographs of the sample of Comparative Example 1.

**Table 1 Tensile Strength of Samples**

| No. | Test Temperature/°C | Size Before Test/mm Thickness *a₀*×Width *b₀* | Original Gauge Length of Sample *L₀*/mm | Maximum Force *Fₘ*/N | Tensile Strength *Rₘ*/MPa |
|---|---|---|---|---|---|
| Example 1 | 25 | 0.322×9.96 | 70 | 1530 | 477 |
| Example 2 | 25 | 0.333×10.21 | 70 | 1478 | 435 |
| Example 3 | 25 | 0.346×10.11 | 70 | 1101 | 315 |
| Example 4 | 25 | 0.341×10.12 | 70 | 1279 | 371 |
| Comparative Example 1 | 25 | 0.332×9.96 | 70 | 822 | 322 |

**Table 2 Elasticity Modulus of Samples**

| No. | Test Temperature/°C | Size Before Test/mm Thickness *a₀*×Width *b₀* | Original Gauge Length of Sample *L₀*/mm | Mass m/g | Dynamic Elasticity Modulus *E_{d}*/GPa |
|---|---|---|---|---|---|
| Example 1 | 25.4 | 5.14×25.19 | 75.34 | 161.288 | 186 |
| Example 2 | 25.4 | 5.17×25.20 | 75.20 | 160.406 | 180 |
| Example 3 | 25.4 | 5.12×25.25 | 75.18 | 159.118 | 181 |
| Example 4 | 25.4 | 5.11×25.19 | 75.25 | 160.062 | 184 |
| Example 5 | 25.4 | 5.17×25.18 | 75.17 | 160.882 | 190 |
| Example 6 | 25.4 | 5.08×25.17 | 75.14 | 160.762 | 189 |
| Comparative Example 1 | 25.4 | 5.10×25.18 | 75.16 | 158.140 | 178 |

Note: In Table 2, the coated samples have a membrane thickness of about 10 µm and the transition layers under the membranes are about 30 µm; apparently, the proportion thereof in the thick tantalum test sample is very small; therefore, the measured data are very close to those of pure tantalum. The test data in Table 2 are substantially close to the elasticity modulus of pure tantalum and cannot represent the elasticity modulus of the surface layers applied on the samples in the present invention; however, the values measured by nanoindentation, e.g., with instrument model TI-950, are capable of representing the variations in the elasticity modulus of different coating processes in the present invention.

The data in Table 1 and Table 2 show that as compared to a tantalum flake not coated with a porous lithium tantalate membrane, a tantalum flake coated with a porous lithium tantalate membrane is greatly improved in tensile strength and elasticity modulus, and particularly has an elasticity modulus much greater than the elasticity modulus of 70 GPa required for preparing dental implants, which is very advantageous.

By comparing the SEM photographs of a tantalum flake coated with a porous lithium tantalate membrane and a tantalum flake not coated with a porous lithium tantalate membrane, it can be shown that the tantalum flake coated with a porous lithium tantalate membrane in the present invention has more openings with a pore diameter in the range of about 1-30 µm, the surfaces of which are rough. These pores and uneven rough surfaces are conducive to cell adhesion and tissue mosaicism and can enhance connection strength between bone implants, such as dental implants, and bone tissues, so that the bone implants can make human biological tissues to be bound with tantalum substrates through porous lithium tantalate membranes and the tantalum substrates can be fully and more tightly bound and integrated with human tissues, such as alveolar bones. However, the tantalum flakes that are not coated with porous lithium tantalate membranes have relatively few pores with smooth and flat surfaces, which is not conducive to close integration of tantalum substrates and human tissues, such as alveolar bones.

In addition, from the SEM photographs, it is apparent that the pores of a tantalum flake coated with a porous lithium tantalate membrane have a size in the smallest dimension in the range of about 10 nm to 1 µm, which is much smaller than the size (which is generally greater than 5 µm) of bacteria. Therefore, the bone implant coated with a porous lithium tantalate membrane in the present invention can well inhibit the entry of bacteria, thereby achieving a certain antibacterial effect.

Figures 9-12 show that a tantalum flake coated with a porous lithium tantalate membrane is white, has an aesthetic appearance and is particularly suitable for use in the preparation of dental implants.

### Example 10

A lithium tantalate membrane layer was formed on a tantalum flake in a molten lithium nitrate:potassium nitrate (in a weight ratio of 1:1) solution, and the process conditions for forming the lithium tantalate membrane layer are shown in Table 3.

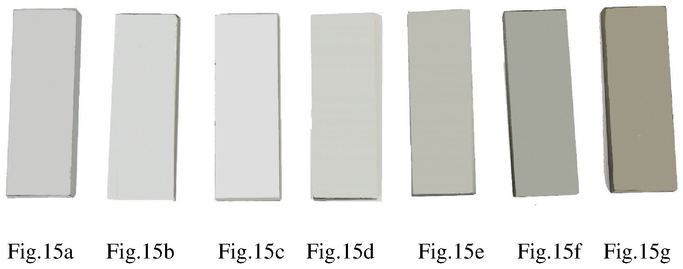

Figure 15a shows an appearance photograph of the sample of Example 10.

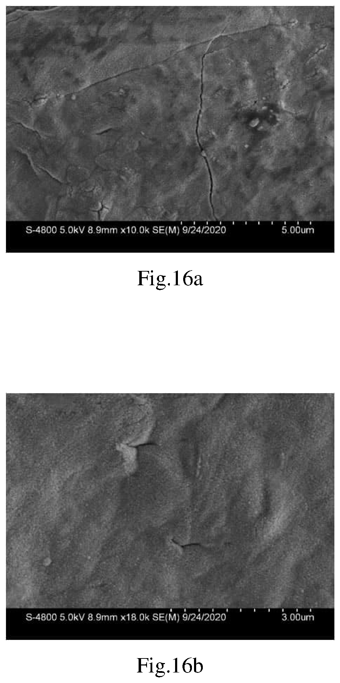

Figures 16a-16b show SEM photographs of the sample of Example 10.

### Example 11

A lithium tantalate membrane layer was formed on a tantalum flake and a tantalum cranium substrate, respectively, in a molten lithium nitrate:potassium nitrate (in a weight ratio of 1:1) solution, and the process conditions for forming the lithium tantalate membrane layer are shown in Table 3.

Figure 15b (a tantalum flake) and Figure 33a (a cranium) show appearance photographs of the sample of Example 11.

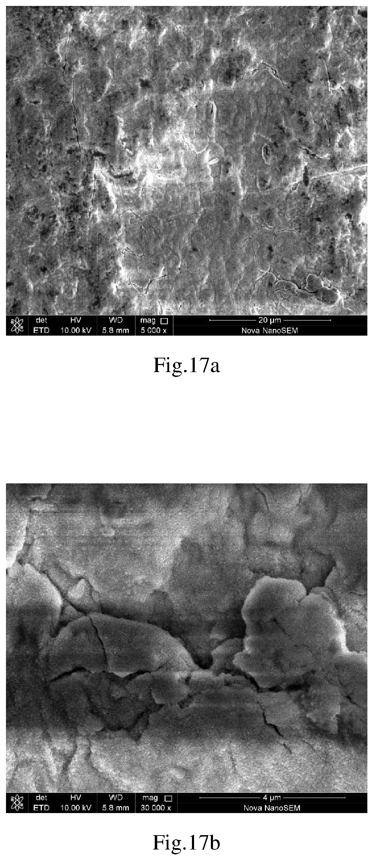

Figures 17a-17b show SEM photographs of the sample of Example 11.

### Example 12

A lithium tantalate membrane layer was formed on a tantalum flake in a molten lithium nitrate:potassium nitrate (in a weight ratio of 1:1) solution, and the process conditions for forming the lithium tantalate membrane layer are shown in Table 3.

Figure 15c shows an appearance photograph of the sample of Example 12.

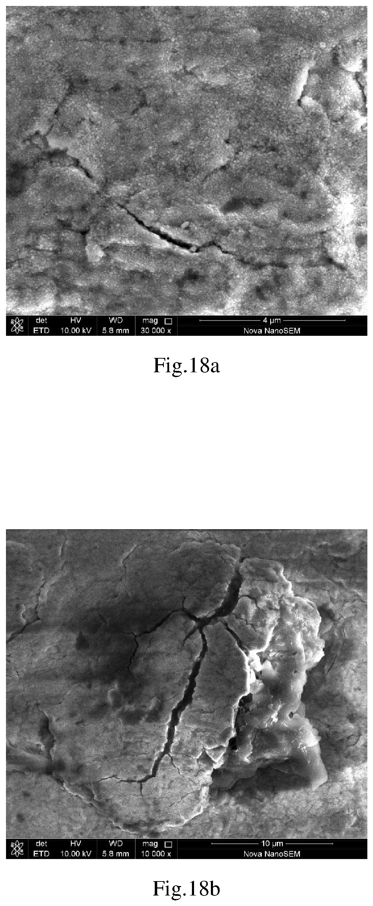

Figures 18a-18b show SEM photographs of the sample of Example 12.

### Example 13

First of all, an anode voltage of 60V was applied on a tantalum flake in a solution having a volume ratio of ethylene glycol:0.01 wt% H₃PO₄ of 2:1 and a temperature of 90°C, and said voltage was held for 1.5 hours, followed by cleaning. After that, a lithium tantalate membrane layer was formed in a molten lithium nitrate:potassium nitrate (in a weight ratio of 1:1) solution, and the process conditions for forming the lithium tantalate membrane layer are shown in Table 3.

Figure 15d shows an appearance photograph of the sample of Example 13.

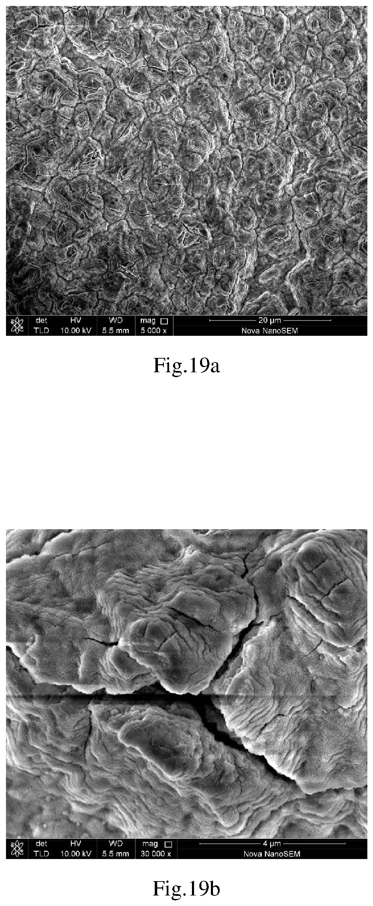

Figures 19a-19b show SEM photographs of the sample of Example 13.

### Example 14

First of all, an anode voltage of 60V was applied on a tantalum flake in a solution having a volume ratio of ethylene glycol:0.01 wt% H₃PO₄ of 2:1 and a temperature of 90°C, and said voltage was held for 1.5 hours, followed by cleaning. After that, a lithium tantalate membrane layer was formed in a molten lithium nitrate:potassium nitrate (in a weight ratio of 1:1) solution, and the process conditions for forming the lithium tantalate membrane layer are shown in Table 3.

Figure 15e shows an appearance photograph of the sample of Example 14.

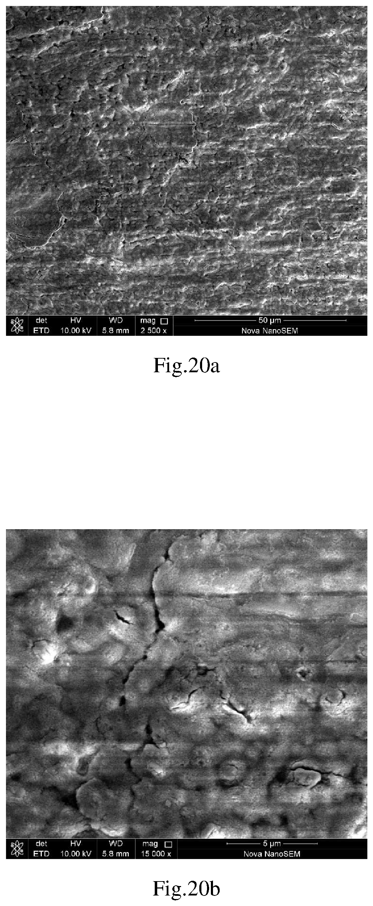

Figures 20a-20b show SEM photographs of the sample of Example 14.

### Example 15

First of all, an anode voltage of 143V was applied on a tantalum flake in a solution having a volume ratio of ethylene glycol:0.01 wt% H₃PO₄ of 2:1 and a temperature of 90°C, and said voltage was held for 1.5 hours, followed by cleaning. After that, a lithium tantalate membrane layer was formed in a molten lithium nitrate:potassium nitrate (in a weight ratio of 1:1) solution, and the process conditions for forming the lithium tantalate membrane layer are shown in Table 3.

Figure 15f shows an appearance photograph of the sample of Example 15.

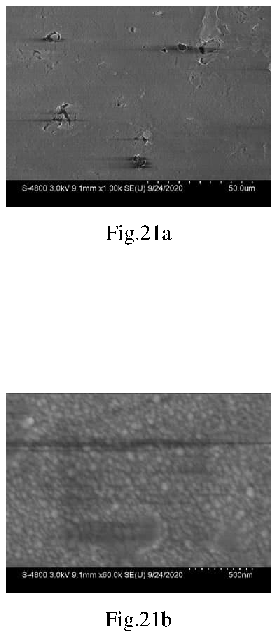

Figures 21a-22b show SEM photographs of the sample of Example 15.

### Example 16

A lithium tantalate membrane layer was formed on a tantalum flake in a molten lithium nitrate:potassium nitrate (in a weight ratio of 1:1) solution, and the process conditions are shown in Table 3.

Figure 15g shows an appearance photograph of the sample of Example 16.

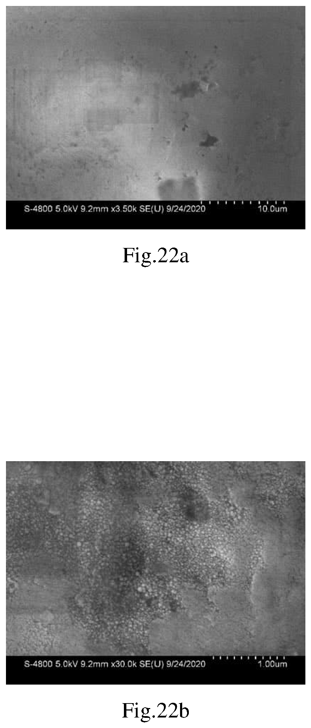

Figures 22a-22b show SEM photographs of the sample of Example 16.

### Example 17

First of all, an anode voltage of 150V was applied on a tantalum flake in a 98% concentrated H₂SO₄ solution at 220°C, and said voltage was held for 30 minutes, followed by cleaning and drying. After that, a lithium tantalate membrane layer was formed in a molten lithium nitrate:potassium nitrate (in a weight ratio of 1:1) solution, and the process conditions for forming the lithium tantalate membrane layer are shown in Table 3.

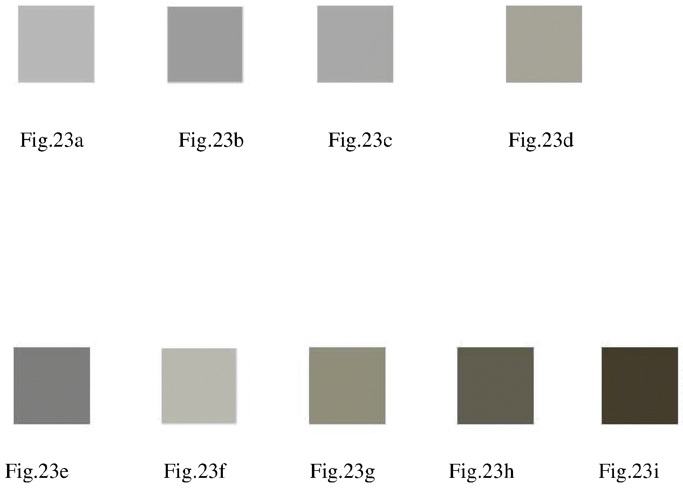

Figure 23a shows an appearance photograph of the sample of Example 17.

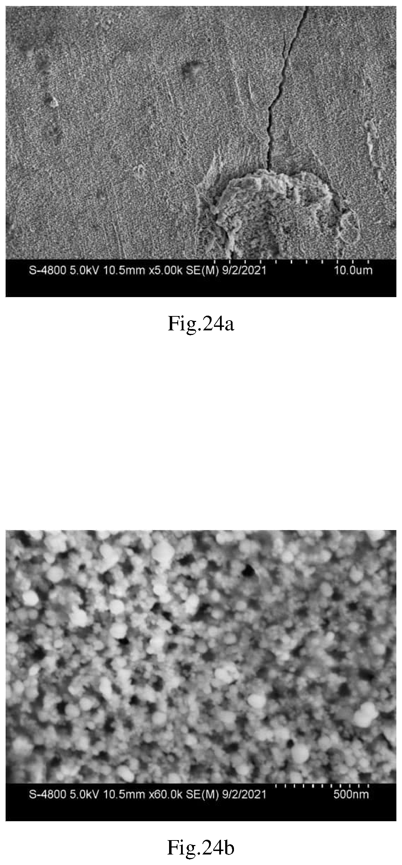

Figures 24a-24b show SEM photographs of the sample of Example 17.

### Example 18

First of all, an anode voltage of 150V was applied on a tantalum flake in a 98% concentrated H₂SO₄ solution at 220°C, and said voltage was held for 30 minutes, followed by cleaning and drying. After that, a lithium tantalate membrane layer was formed in a molten lithium nitrate:potassium nitrate (in a weight ratio of 1:1) solution, and the process conditions for forming the lithium tantalate membrane layer are shown in Table 3.

Figure 23b shows an appearance photograph of the sample of Example 18.

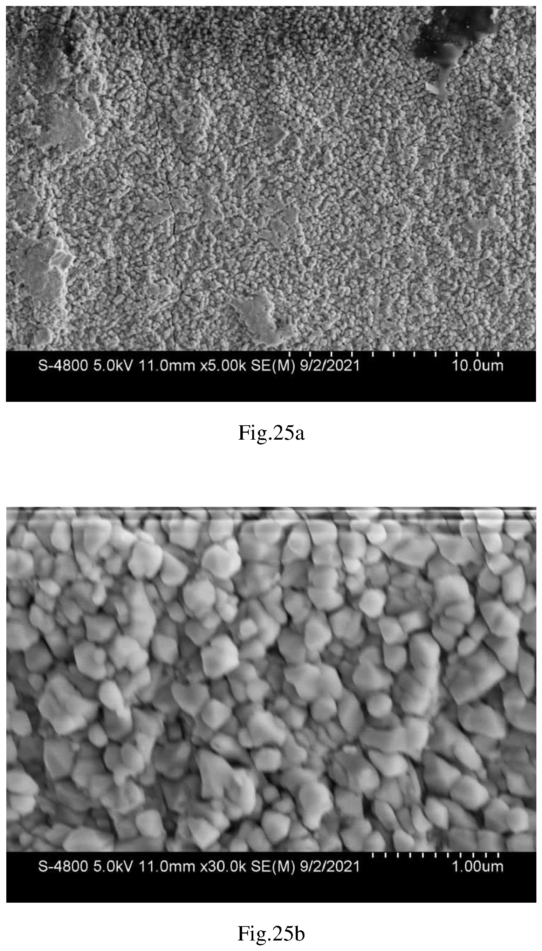

Figures 25a-25b show SEM photographs of the sample of Example 18.

### Example 19

First of all, an anode voltage of 150V was applied on a tantalum flake in a 98% concentrated H₂SO₄ solution at 220°C, and said voltage was held for 30 minutes, followed by cleaning and drying. After that, a lithium tantalate membrane layer was formed in a molten lithium nitrate:potassium nitrate (in a weight ratio of 1:1) solution, and the process conditions for forming the lithium tantalate membrane layer are shown in Table 3.

Figure 23c shows an appearance photograph of the sample of Example 19.

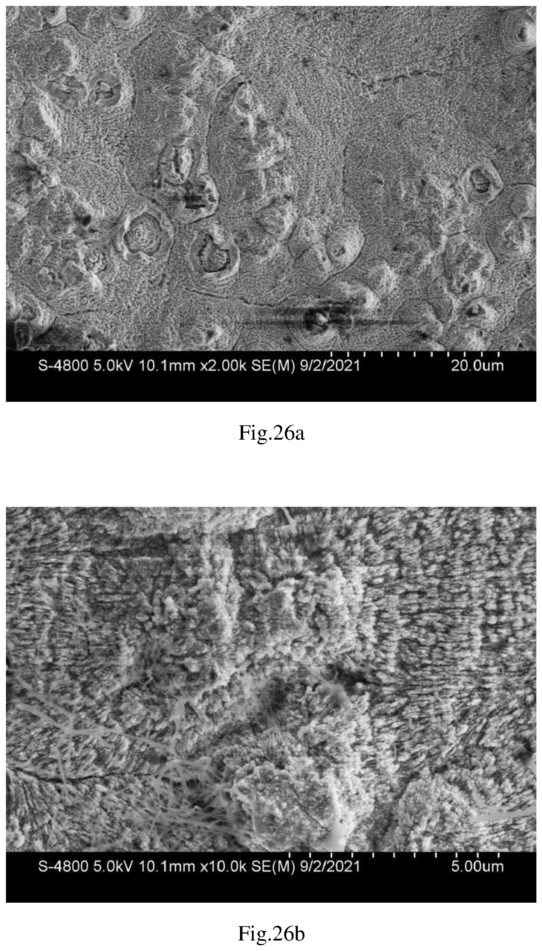

Figures 26a-26b show SEM photographs of the sample of Example 19.

### Example 20

First of all, an anode voltage of 82V was applied on a tantalum flake in a 98% concentrated H₂SO₄ solution at 220°C, and said voltage was held for 30 minutes, followed by cleaning and drying. After that, a lithium tantalate membrane layer was formed in a molten lithium nitrate:potassium nitrate (in a weight ratio of 1:1) solution, and the process conditions for forming the lithium tantalate membrane layer are shown in Table 3.

Figure 23d shows an appearance photograph of the sample of Example 20.

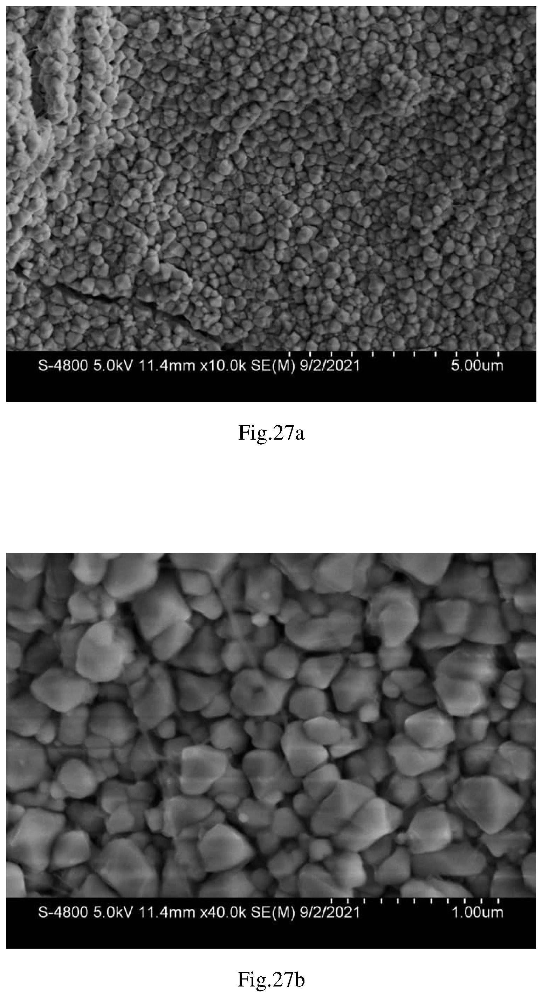

Figures 27a-27b show SEM photographs of the sample of Example 20.

### Example 21

First of all, an anode voltage of 82V was applied on a tantalum flake in a 98% concentrated H₂SO₄ solution at 210°C, and said voltage was held for 30 minutes, followed by cleaning and drying. After that, a lithium tantalate membrane layer was formed in a molten lithium nitrate:potassium nitrate (in a weight ratio of 1:1) solution, and the process conditions for forming the lithium tantalate membrane layer are shown in Table 3.

Figure 23e shows an appearance photograph of the sample of Example 21.

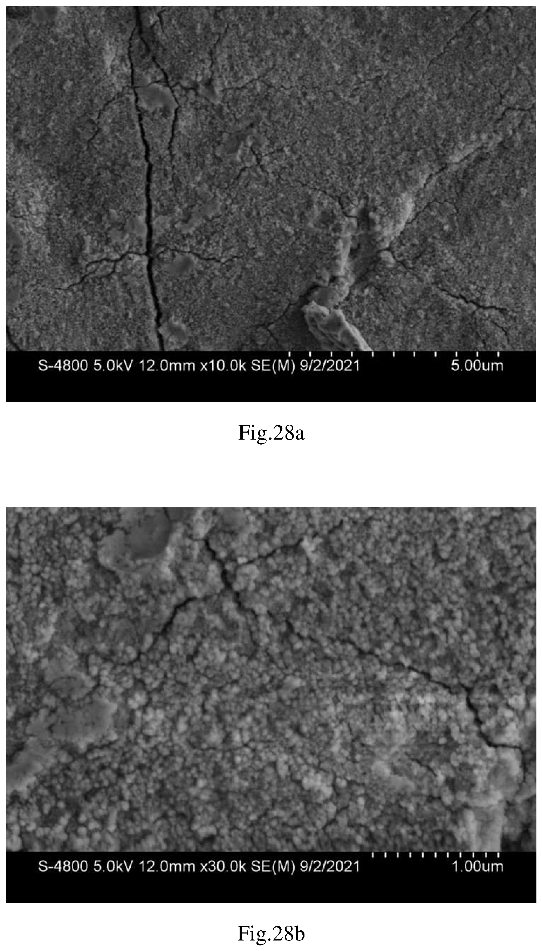

Figures 28a-28b show SEM photographs of the sample of Example 21.

### Example 22

First of all, an anode voltage of 82V was applied on a tantalum flake in a 98% concentrated H₂SO₄ solution at 210°C, and said voltage was held for 30 minutes, followed by cleaning and drying. After that, a lithium tantalate membrane layer was formed in a molten lithium nitrate:potassium nitrate (in a weight ratio of 1:1) solution, and the process conditions for forming the lithium tantalate membrane layer are shown in Table 3.

Figure 23f shows an appearance photograph of the sample of Example 22.

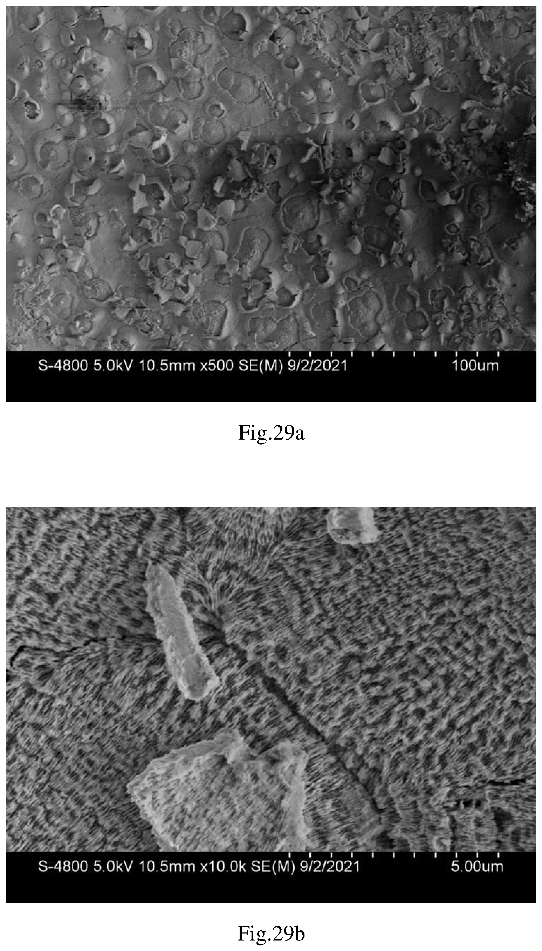

Figures 29a-29b show SEM photographs of the sample of Example 22.

### Example 23

First of all, an anode voltage of 82V was applied on a tantalum flake in a 98% concentrated H₂SO₄ solution at 210°C, and said voltage was held for 30 minutes, followed by cleaning and drying. After that, a lithium tantalate membrane layer was formed in a molten lithium nitrate:potassium nitrate (in a weight ratio of 1:1) solution, and the process conditions for forming the lithium tantalate membrane layer are shown in Table 3.

Figure 23g shows an appearance photograph of the sample of Example 23.

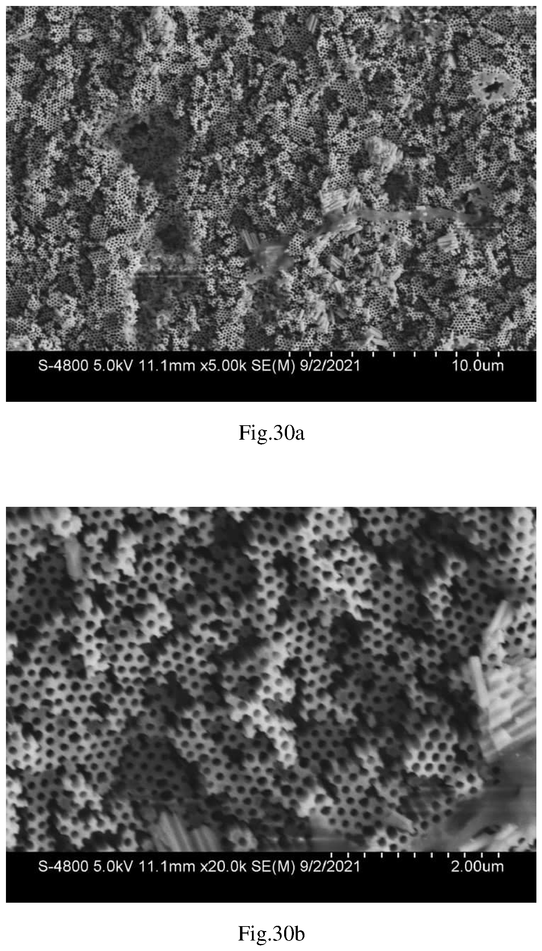

Figures 30a-30b show SEM photographs of the sample of Example 23.

### Example 24

A tantalum flake was anodized in a 98% concentrated H₂SO₄ solution at 220°C, and an anode voltage of 150V was applied, which voltage was maintained for 30 minutes. See Table 3.

Figure 23h shows an appearance photograph of the sample of Example 24.

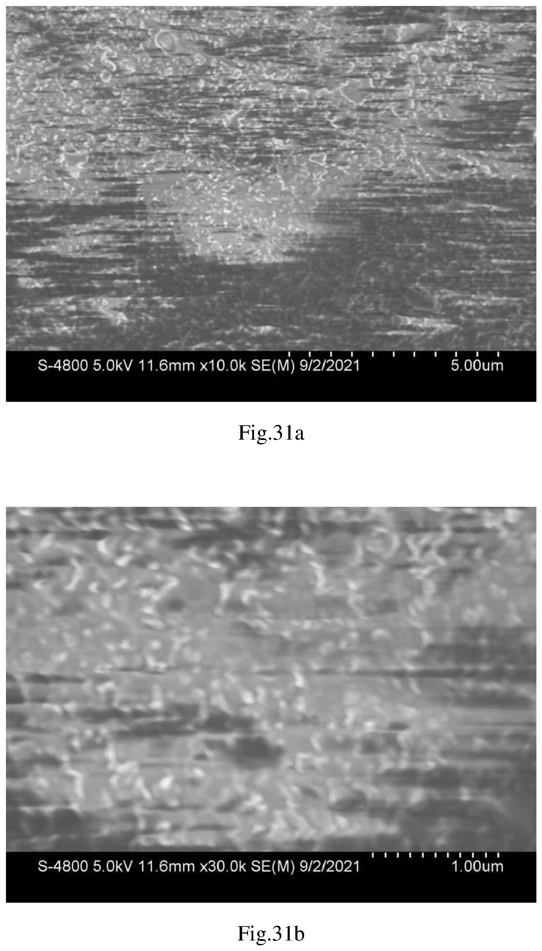

Figures 31a-31b show SEM photographs of the sample of Example 24.

### Example 25

A titanium flake was anodized in a molten lithium nitrate:potassium nitrate (in a weight ratio of 1:1) solution to form a titanium oxide membrane layer, and the process conditions for forming the titanium oxide membrane layer are shown in Table 3.

Figure 23i shows an appearance photograph of the sample of Example 25.

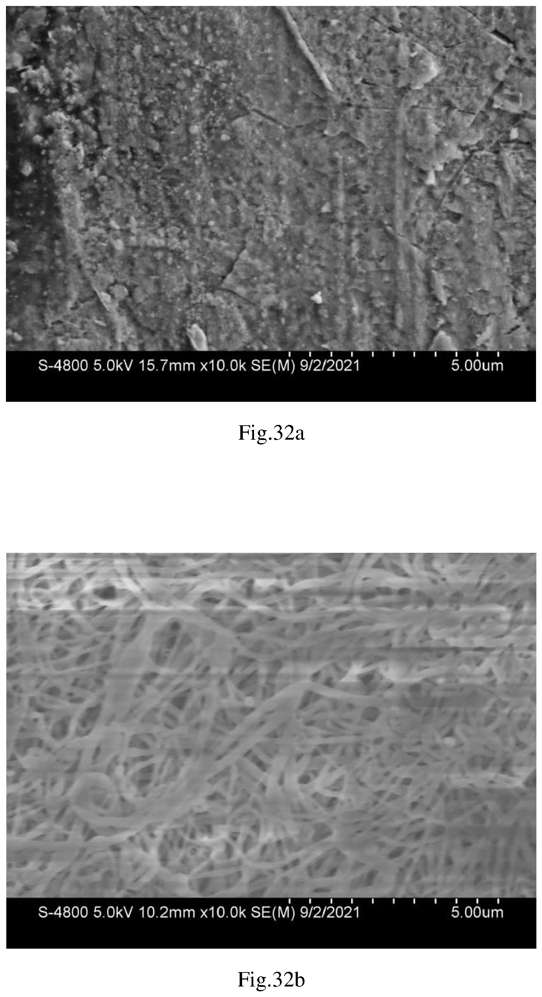

Figures 32a-32b show SEM photographs of the sample of Example 25.

The data in Table 3 show that the tantalum complexes coated with a porous lithium tantalate membrane have a suitable elasticity modulus and are suitable, for example, for the preparation of cranium (skull) implants.

By comparing the SEM photographs of a tantalum flake coated with a porous lithium tantalate membrane and a tantalum flake not coated with a porous lithium tantalate membrane, it can be shown that the tantalum flake coated with a porous lithium tantalate membrane in the present invention has more openings with a pore diameter in the range of about 0.1-30 µm and even honeycomb openings, the surfaces of which are rough. These pores and uneven rough surfaces are conducive to cell adhesion and tissue mosaicism and can enhance connection strength between bone implants, such as dental implants, and bone tissues, such as craniums (skulls), so that the bone implants can make human biological tissues to be bound with tantalum substrates through porous lithium tantalate membranes and the tantalum substrates can be fully and more tightly bound and integrated with human tissues. However, the tantalum flakes that are not coated with porous lithium tantalate membranes have relatively few pores with smooth and flat surfaces, which is not conducive to close integration of tantalum substrates and human tissues, such as alveolar bones and craniums (skulls).

The titanium flake with a titanium oxide membrane layer prepared in Example 25 exhibits a suitable elasticity modulus and its SEM photographs show that the membrane layer has a fibrous porous interactive network (see Figure 32b), which is conducive to cell adhesion and tissue mosaicism and can enhance connection strength between bone implants and bone tissues (such as craniums (skulls)), so that the bone implants can make human biological tissues to be bound with titanium substrates through porous titanium oxide membrane layers and the titanium substrates can be fully and more tightly bound and integrated with human tissues.

In addition, from the SEM photographs, it is apparent that the pores of a tantalum flake coated with a porous lithium tantalate membrane have a size in the smallest dimension in the range of about 10 nm to 1 µm, which is much smaller than the size (which is generally greater than 5 µm) of bacteria. Therefore, the bone implant coated with a porous lithium tantalate membrane in the present invention can well inhibit the entry of bacteria, thereby achieving a certain antibacterial effect.

### Example 26

A lithium tantalate-lithium niobate mixture membrane layer was formed on a tantalum-niobium alloy flake in a molten lithium nitrate:potassium nitrate (in a weight ratio of 1:1) solution, and the process conditions for forming the lithium tantalate-lithium niobate mixture membrane layer are shown in Table 3.

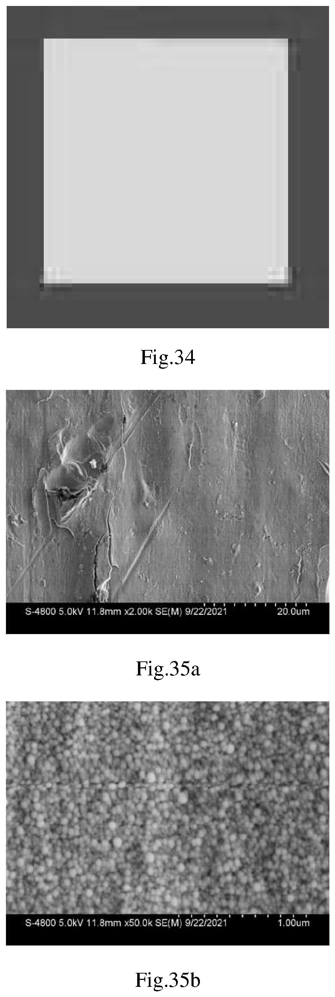

Figure 34 shows an appearance photograph of the sample of Example 26.

Figures 35a-35b show SEM photographs of the sample of Example 26.

### Example 27

First of all, an anode voltage of 143V was applied on a tantalum-niobium alloy flake in a solution having a volume ratio of ethylene glycol:0.01 wt% H₃PO₄ of 2:1 and a temperature of 90°C, and said voltage was held for 1.5 hours, followed by cleaning. After that, a lithium tantalate-lithium niobate mixture membrane layer was formed in a molten lithium nitrate:potassium nitrate (in a weight ratio of 1:1) solution, and the process conditions for forming the lithium tantalate-lithium niobate mixture membrane layer are shown in Table 3.

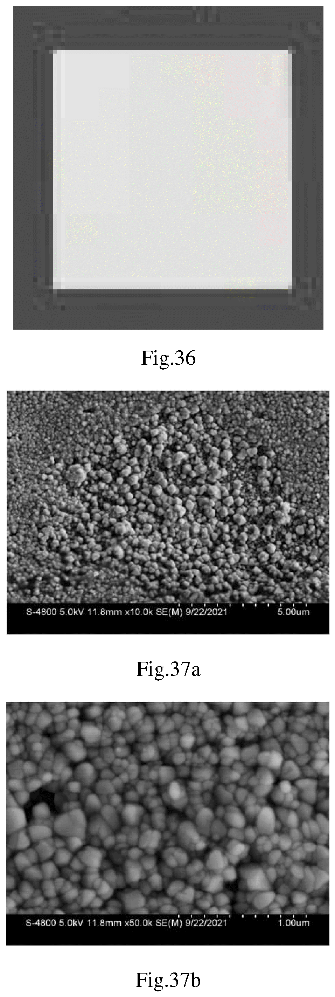

Figure 36 shows an appearance photograph of the sample of Example 27.

Figures 37a-37b show SEM photographs of the sample of Example 27.

### Example 28

A lithium niobate membrane layer was formed on a niobium flake in a molten lithium nitrate:potassium nitrate (in a weight ratio of 1:1) solution, and the process conditions for forming the lithium niobate membrane layer are shown in Table 3.

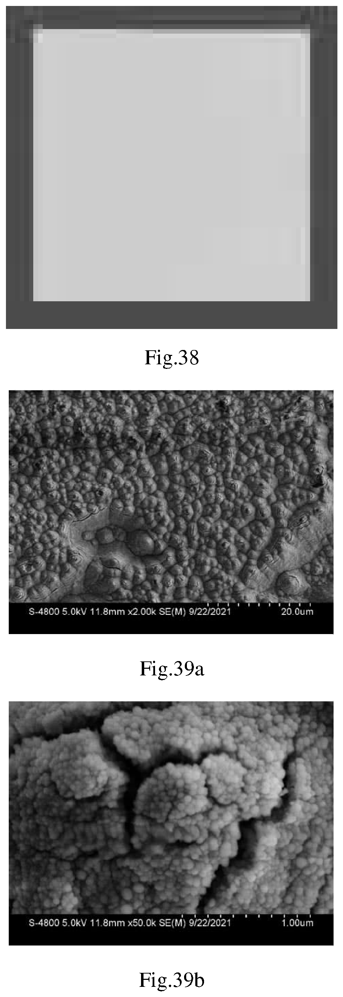

Figure 38 shows an appearance photograph of the sample of Example 28.

Figures 39a-39b show SEM photographs of the sample of Example 28.

### Example 29

A lithium niobate membrane layer was formed on a niobium flake and a niobium cranium substrate in a molten lithium nitrate:potassium nitrate (in a weight ratio of 1:1) solution, and the process conditions for forming the lithium niobate membrane layer are shown in Table 3.

Figure 40 (a niobium flake) and Figure 41 (a niobium cranium) show appearance photographs of the sample of Example 29.

Figures 42a-42b show SEM photographs of the sample of Example 29.

### Example 30

A lithium niobate membrane layer was formed on a niobium flake in a molten lithium nitrate:potassium nitrate (in a weight ratio of 1:1) solution, and the process conditions for forming the lithium niobate membrane layer are shown in Table 3.

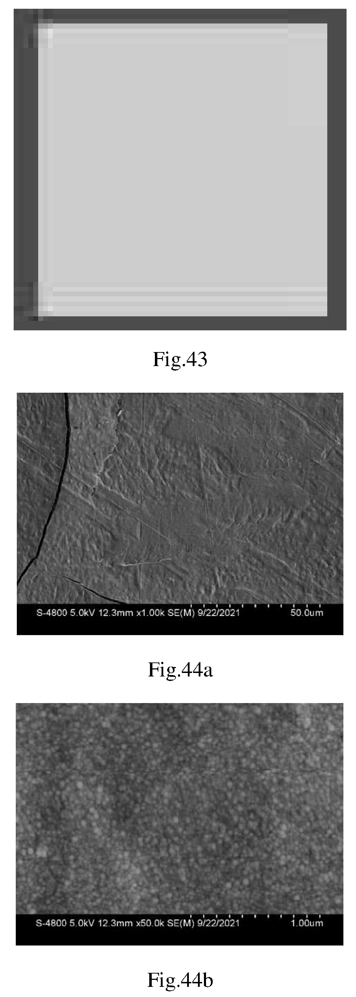

Figure 43 shows an appearance photograph of the sample of Example 30.

Figures 44a-44b show SEM photographs of the sample of Example 30.

**Table 3 Preparation and Elasticity Modulus of Samples (measured by nanoindenter TI-950, NHT, from Hysitron, US)**

| No. | Test Temperature (°C) | Test Voltage (V) | Test Time (min) | Elasticity Modulus (Ed/GPa) | Specification (mm) |
|---|---|---|---|---|---|
| Example 10 | 570 | 10 | 30 | 134.164 | 80*25*5 |
| Example 11 | 580 | 10 | 25 | 43.266 | 80*25*5 |
| Example 12 | 598 | 10 | 10 | 70.108 | 80*25*5 |
| Example 13 | 598 | 10 | 8 | 105.314 | 80*25*5 |
| Example 14 | 598 | 10 | 10 | 87.743 | 80*25*5 |
| Example 15 | 482 | 28 | 390 | 74.678 | 80*25*5 |
| Example 16 | 440 | 33 | 720 | 119.325 | 80*25*5 |
| Example 17 | 482 | 28 | 390 | 93.23964 | 10*10*0.28 |
| Example 18 | 580 | 10 | 25 | 112.601 | 10*10*0.28 |
| Example 19 | 598 | 10 | 10 | 134.1445 | 10*10*0.28 |
| Example 20 | 580 | 10 | 25 | 17.30241 | 10*10*0.28 |
| Example 21 | 482 | 20 | 390 | 115.0177 | 10*10*0.28 |
| Example 22 | 482 | 8 | 390 | 83.64055 | 10*10*0.28 |
| Example 23 | 210 | 82 | 30 | 13.34679 | 10*10*0.28 |
| Example 24 | 220 | 150 | 30 | 24.87508 | 10*10*0.28 |
| Example 25 | 580 | 10 | 110 | 65.62898 | 10*10*0.28 |
| Example 26 | 482 | 28 | 300 | 64.466 | 10*10*0.28 |
| Example 27 | 482 | 28 | 180 | 50.504 | 10*10*0.28 |
| Example 28 | 460 | 28 | 0.083 | 70.108 | 10*10*0.28 |
| Example 29 | 460 | 10 | 20 | 48.356 | 10*10*0.28 |
| Example 30 | 460 | 20 | 5 | 54.295 | 10*10*0.05 |

The elasticity moduli in Table 3 are measured by nanoindenter TI-950, NHT, from Hysitron, US.

The data in Table 3 show that the complexes coated with a porous lithium tantalate-lithium niobate mixture membrane and the complexes with a porous lithium niobate membrane have a suitable elasticity modulus and are suitable, for example, for the preparation of cranium (skull) implants.

Although the specific embodiments of the present invention have been shown and described, it should be understood that other modifications, substitutions and alternatives are known to those skilled in the art. Such modifications, substitutions and alternatives can be made without departing from the spirit and scope of the present invention, which should be determined by the attached claims. The various features of the present invention are described in the attached claims.

## Claims

1. A method for preparing a bone implant, comprising:
(1) providing a substrate; and
(2) forming a porous membrane on the substrate,
wherein one of these features applies:
(i) the substrate is a tantalum substrate and the porous membrane is a porous lithium tantalate membrane, which contains a tantalum oxide,
(ii) the substrate is a niobium substrate and the porous membrane is a porous lithium niobate membrane, which contains a niobium oxide or
(iii) the substrate is a tantalum-niobium alloy substrate and the porous membrane is a porous lithium tantalate-lithium niobate mixture membrane, which contains a tantalum oxide and a niobium oxide,
wherein in step (2), a tantalum substrate, a niobium substrate or a tantalum-niobium alloy substrate is placed in an oxygen-containing inorganic lithium salt or a mixed melt of an oxygen-containing inorganic lithium salt and lithium hydroxide, or a mixed molten liquid of a salt and lithium hydroxide or a mixed molten liquid of a lithium salt and an oxygen-containing salt, at a temperature of 250°C to 650°C, and an anode voltage of 1 to 66V is applied, which voltage is maintained for 0.01 to 200 hours, with a boosting current density of 1 to 1000 mA/cm², to form a membrane layer containing a lithium-containing compound.

2. The method according to claim 1, wherein in step (2), a tantalum substrate, a niobium substrate or a tantalum-niobium alloy substrate is placed in an oxygen-containing inorganic lithium salt or a mixed melt of an oxygen-containing inorganic lithium salt and lithium hydroxide, or a mixed molten liquid of a salt and lithium hydroxide or a mixed molten liquid of a lithium salt and an oxygen-containing salt, at a temperature of 440°C to 600°C, and an anode voltage of 10 to 30V is applied, which voltage is maintained for 5 minutes to 10 hours, with a boosting current density of 5 to 20 mA/cm², to form a membrane layer containing a lithium-containing compound.

3. The method according to claim 1, wherein an ultrasonic generator is placed in the mixed melt or the mixed molten liquid.

4. The method according to claim 1, wherein a tantalum substrate, a niobium substrate or a tantalum-niobium alloy substrate is nitrided or carburized.

5. A method for preparing a dental implant having pores, comprising:
1) providing a dental implant niobium blank and designing punching parameters,
i.e., determining the size of the cylindrical or conical implant, including the outer diameter of the cylinder and the diameter of the inner pores, expanding the cylinder with pores into a planar view, and designing and determining the size of the pores, the pore spacing or the pore distribution, as well as the punching depth of the pores and the shape of the pores, i.e., a straight pore or a tapered pore,
2) punching pores,
3) polishing,
4) welding a punched and polished niobium flake with argon arc welding or laser welding into the originally designed cylindrical or conical implant, and performing necessary repairs and processing internal threads;
5) performing the surface treatment on the porous implant and an abutment by an electrochemical method to form a complex porous membrane corresponding to the implant metal and obtain a coated dental implant with pores, wherein the porous membrane is a porous lithium niobate membrane.
